# EUROPÄISCHE PATENTSCHRIFT

(11) **EP 1 453 852 B1**
(45) Veröffentlichungstag und Bekanntmachung des Hinweises auf die Patenterteilung: **17.01.2007**
(21) Anmeldenummer: 02787942.8
(22) Anmeldetag: 12.12.2002
(51) Int. Cl.: C07K 5/06, C07C 311/13, C07C 311/47

(54) **SELEKTIVE ARYLGUANIDINPEPTIDE ALS UROKINASEINHIBITOREN**
SELECTIVE UROKINASE INHIBITORS
INHIBITEURS SELECTIFS D'UROKINASE

(30) Priorität: 12.12.2001 DE 10161062; 02.09.2002 DE 10240452
(43) Veröffentlichungstag der Anmeldung: 08.09.2004
(73) Patentinhaber: Wilex AG, 81675 München (DE)
(72) Erfinder: SPERL, Stefan, 81549 München (DE)
(74) Vertreter: Dey, Michael
(86) Internationale Anmeldenummer: PCT/EP2002/014157
(87) Internationale Veröffentlichungsnummer: WO 2003/053999

(56) Entgegenhaltungen:
- WO-A-00/05245
- WO-A-01/14324
- WO-A-01/70204
- WO-A-01/96286
- WO-A-01/96366
- WO-A-02/14349
- US-A- 5 726 159
- S SPERL ET AL. : "(4-AMINOMETHYL)PHENYLGUANIDINE DERIVATIVES AS NONPEPTIDIC HIGHLY SELECTIVE INHIBITORS OF HUMAN UROKINASE" PROCEEDINGS OF THE NATIONAL ACADEMY OF SCIENCES OF USA., Bd. 97, Nr. 10, 9. Mai 2000 (2000-05-09), Seiten 5113-5118, XP002169711 NATIONAL ACADEMY OF SCIENCE. WASHINGTON., US ISSN: 0027-8424

## Beschreibung

Die vorliegende Erfindung betrifft neue selektive Inhibitoren des Urokinase-Plasminogenaktivators (uPA, EC 3.4.21.31) sowie deren Verwendung als therapeutische Wirkstoffe zur Behandlung von Urokinase assoziierten Erkrankungen, wie z.B. maligne Tumore und Metastasierung. Die Erfindung betrifft insbesondere neue hochselektive und hochaktive Inhibitoren des Urokinase-Plasminogenaktivators (uPA, EC 3.4.21.31) vom Arylguanidintyp.

Der Plasminogenaktivator von Urokinase-Typ (uPA) spielt eine Schlüsselrolle bei der Tumorinvasion und Metastasenbildung (Schmitt et al., J. Obst. Gyn. 21 (1995), 151-165). uPA wird in verschiedenen Arten von Tumorzellen überexprimiert (Kwaan, Cancer Metastasis Rev. 11 (1992), 291-311) und bindet an den Tumor-assoziierten uPA-Rezeptor (uPA-R), wo die Aktivierung von Plasminogen zu Plasmin stattfindet. Plasmin ist in der Lage, verschiedene Komponenten der extrazellulären Matrix (ECM) wie Fibronectin, Laminin und Kollagen Typ IV abzubauen. Es aktiviert auch einige andere ECM-abbauende Enzyme, insbesondere Matrix-Metalloproteinasen. Hohe Mengen an Tumor-assoziiertem uPA korrelieren mit einem höheren Metastasierungsrisiko für Krebspatienten (Stephens et al., Breast Cancer Res. & Treat. 52 (1998), 99-111). Eine Hemmung der proteolytischen Aktivität von uPA ist daher ein guter Ansatzpunkt für eine anti-metastatische Therapie.

Ein gemeinsames Merkmal vieler bekannter synthetischer uPA-Inhibitoren ist ein basischer Rest, der Amidino- oder Guanidino-Gruppen enthält, und an Asp¹⁸⁹ in der S1-Spezifitätstasche von uPA binden kann und dort als Arginin-Mimetikum wirkt (Spraggon et al., Structure 3 (1995), 681-691).

Die meisten der bekannten Inhibitoren sind jedoch nicht selektiv für uPA, sondern hemmen auch andere Serinproteasen wie Trypsin, Thrombin, Plasmin oder Gewebs-Plasminogenaktivator (tPA).

p-Aminobenzamidin ist ein moderat selektiver uPA-Inhibitor mit einer Hemmkonstante von 82 µM. Billstroem et al. (Int. J. Cancer 61 (1995), 542-547) konnten eine deutliche Abnahme der Wachstumsrate von DU145 Tumoren (eine Prostata-Adenokarzinom-Zellinie) in SCID Mäusen bei oraler Verabreichung in einer Tagesdosis von 125 bis 250 mg p-Aminobenzamidin/kg/Tag zeigen. Die Nebenwirkungen waren vernachlässigbar gering.

Einige monosubstituierte Phenylguanidine haben sich als wirksame und selektive uPA Inhibitoren in vitro erwiesen. Diese kleinen Moleküle zeigen Inhibierungskonstanten im Mikromolarbereich, sie binden jedoch nur in der S1 Tasche von uPA (Yang et al., J. Med. Chem. 33 (1990), 2956-2961). Biologische Untersuchungen mit diesen Verbindungen wurden nicht durchgeführt.

Das Diuretikum Amilorid ist ein selektiver uPA-Inhibitor (Ki, uPA = 7 µM), der die Bildung von Lungenmetastasen nach i.v. Inokulation von Ratten-Brustadenokarzinomzellen verhindert (Kellen et al., Anticancer Res. 8 (1988), 1373-1376). Einige Derivate von 3-Amidino-phenylalanin haben sich ebenfalls als wirksame Inhibitoren von Serinproteasen erwiesen, diese Verbindungen weisen jedoch im allgemeinen nur eine geringe Selektivität für uPA auf (Stürzebecher et al., J. Med. Chem. 40 (1997), 3091-3099; Stürzebecher et al., J. Enzyme Inhib. 9 (1995), 87-99).

Bekannte uPA-Inhibitoren sind Derivate von Benzo[b]thiophen-2-carboxamidin (B428 und B623: Kᵢ, uPA = 0,32 bzw. 0,07 µM; US-Patent 5,340,833). Räbbani et al. (Int. J. Cancer 63 (1995), 840-845) sowie Xing et al. (Cancer Res. 57 (1997), 3585-3593) konnten nach Verabreichung von 4-lod-benzo[b]-thiophen-2-carboxamidin (B428) eine Abnahme des Tumorwachstums und der Metastasenbildung in einem syngenen Modell für Ratten-Prostatakarzinom bzw. Maus-Mammakarzinom zeigen. Letztere Untersuchungen zeigten eine weitere Abnahme des Primärtumorwachstums bei gemeinsamer Verabreichung von B428 mit dem Antiöstrogen Tamoxifen.

Die deutsche Patentanmeldung 199 40 389.9 schlägt die Verwendung von Arylguanidin- und insbesondere Phenylguanidin-Derivaten als selektive uPA-Inhibitoren vor. Diese Verbindungen enthalten einen weiteren Substituenten am aromatischen Ringsystem, vorzugsweise in Para-Position zur Guanidingruppe, der eine gegebenenfalls substituierte Methylengruppe gefolgt von Wasserstoffdonor/Akzeptorfunktionalitäten enthält. Aufgrund dieses Substitutionsmusters weisen die Verbindungen eine besonders hohe Wirksamkeit und Selektivität für uPA auf. Von diesen Verbindungen wird angenommen, dass sie als Arginin-Mimetikum mit dem Aminosäurerest Asp¹⁸⁹ in der S1-Tasche von uPA wechselwirken und eine Wechselwirkung mit der S2- und/oder S3-Tasche von uPA eingehen können.

Die deutsche Patentanmeldung 100 13 715.6 beschreibt weitere ArylGuanidin-Derivate, die eine noch spezifischere Wechselwirkung mit uPA, insbesondere mit den Aminosäureresten Gln¹⁹² und/oder Ser²¹⁴ eingehen können. Diese Verbindungen enthalten neben der Guanidin-Gruppe einen weiteren Substituenten am aromatischen Ringsystem, der eine gegebenenfalls substituierte Methylengruppe gefolgt von einer Wasserstoffdonor-, einer Wasserstoffakzeptor- und wiederum einer Wasserstoffdonorfunktionalität enthält.

Die internationale Patentanmeldung WO 00/04954 beschreibt Arylamidin-Derivate, insbesondere Amidinophenylalanin-Derivate als Urokinaseinhibitoren.

Eine Aufgabe der vorliegenden Erfindung war die Bereitstellung neuer hochselektiver und hochaktiver Inhibitoren des Urokinase-Plasminogenaktivators.

Diese Aufgabe wird erfindungsgemäß gelöst durch die Verwendung der Verbindungen N-[(4-Guanidino-benzylcarbamoyl)-methyl]-3-hydroxy-2-phenylmethansulfonylaminopropionamid(WX-508),Benzylsulfonyl-(D)-Ser-Ala-(4-Guanidinobenzyl)amid Hydrochlorid (WX-532), Benzylsulfonyl-(D)-Ser-N-Me-Gly-(4-Guanidinobenzylamid (WX-538), 4-Chlorbenzylsulfonyl-(d)-Ser-N-Me-Ala-(4-Guanidinobenzyl)amid (WX-582). 4-Chlorbenzyl-sulfonyl-(D)-Ser-Gly-(4-Guanidinobenzyl)amid Hydrochlorid (WXC-340), 3-Chlorbenzyl-sulfonyl-(D)-Ser-Gly-(4-Guanidinobenzyl)amid Hydrochlorid (WXC-318), 3-Nitrobenzyl-sulfonyl-(D)-Ser-Gly-(4-Guanidinobenzyl)amid Hydrochlorid (WXC-316), N-(4-Guanidino-benzyl)-2-(3-hydroxy-2-phenylmethan-sulfonylamino-propionylamino)-4-phenyl-butyramid Hydrochlorid (WX-550), Benzylsulfonyl-(D)-Ser-Ala-(4-Guanidinobenzyl)amid Hydrochlorid (WX-532) sowie der Verbindungen Bz-SO₂-(D)-Ser-(Aza-Gly)-4-Guanidino-benzylamid Hydrochlorid (WX-544) und N-[2-(4-Guanidino-benzolsulfonylamino)-ethyl]-3-hydroxy-2-phenylmethansulfonylamino-propionamid Hydrochlorid (WX-568) oder Salzen dieser Verbindungen zur Herstellung eines Mittels zur Hemmung des Urokinase-Plasminogenaktivators.

Die Verbindungen können als Salze, vorzugsweise als physiologisch verträgliche Säuresalze, z.B. als Salze von Mineralsäuren, besonders bevorzugt als Hydrochloride oder als Salze von geeigneten organischen Säuren vorliegen. Die Guanidiniumgruppe kann gegebenenfalls Schutzfunktionen tragen, die vorzugsweise unter physiologischen Bedingungen abspaltbar sind. Die Verbindungen können als optisch reine Verbindungen oder als Gemische von Enantiomeren oder/und Diastereoisomeren vorliegen.

Die erfindungsgemäßen Urokinaseinhibitoren können gegebenenfalls zusammen mit geeigneten pharmazeutischen Hilfs- oder Trägerstoffen zur Herstellung von Arzneimitteln oder in der Diagnostik verwendet werden. Dabei ist eine Verabreichung in Kombination mit anderen Wirkstoffen, z.B. anderen Urokinaseinhibitoren, wie etwa Antikörpern oder/und Peptiden, aber auch mit Chemotherapeutika und Zytostatika oder/und zytostatischen Wirkstoffen möglich.

Die Arzneimittel können bei Menschen und Tieren topisch, oral, rektal oder parenteral, z.B. intravenös, subkutan, intramuskulär, intraperitoneal, sublingual, nasal oder/und inhalativ, z.B. in Form von Tabletten, Dragees, Kapseln, Pellets, Suppositorien, Lösungen, Emulsionen, Suspensionen, Liposomen, Inhalationssprays oder transdermalen Systemen, wie Pflastern, verabreicht werden.

Die erfindungsgemäßen Verbindungen sind zur Bekämpfung von Krankheiten geeignet, die mit einer pathologischen Überexpression von uPA oder/und Urokinase-Plasminogenaktivatorrezeptor (uPAR) assoziiert sind. Sie sind beispielsweise in der Lage, hocheffizient das Wachstum oder/und die Ausbreitung der malignen Tumoren sowie die Metastasierung von Tumoren zu hemmen. Dabei können die uPA-Inhibitoren gegebenenfalls zusammen mit anderen Tumormitteln oder mit anderen Behandlungsarten, z.B. Bestrahlung oder chirurgischen Eingriffen, eingesetzt werden. Weiterhin sind die erfindungsgemäßen Inhibitoren auch für andere uPA-assoziierte oder/und uPAR-assoziierte Erkrankungen wirksam.

Erfindungsgemäße uPA-Inhibitoren sind vorzugsweise dadurch gekennzeichnet, daß sie mindestens einen zweifach, vorzugsweise mindestens einen fünffach und besonders bevorzugt einen mindestens 10-und bis zu 1.000-fach geringeren Kᵢ-Wert für uPA gegenüber tPA, Plasmin oder/und Thrombin aufweisen. Weiterhin ist bemerkenswert, dass die erfindungsgemäßen Verbindungen die Blutgerinnung nur geringfügig beeinflussen, da sie für eine effektive Hemmung von Thrombin, Plasmin und Faktor Xa zu hohe Kᵢ-Werte haben.

Die erfindungsgemäßen Substanzen können in Kombination mit physiologisch wirksamen Substanzen eingesetzt werden, z.B. mit Radiomarkierungen oder mit zytotoxischen Mitteln, z.B. Chemotherapeutika wie cis-Platin oder 5-Fluor-uracil, oder Peptiden. Weiterhin können die Substanzen auch in die Membran von Trägervesikeln, z.B. Liposomen, eingebaut werden oder/und zusammen mit in den Trägervesikeln eingeschlossenen Wirksubstanzen, z.B. zytotoxischen Mitteln, wie etwa Doxorubicin, verabreicht werden.

Durch die Erfindung wird ein Verfahren zur Urokinasehemmung bei Lebewesen, insbesondere bei Menschen, durch Verabreichung einer wirksamen Menge mindestens einer erfindungsgemäßen Verbindung bereitgestellt. Die Dosierung der Verbindung liegt üblicherweise im Bereich von 0,01 bis 100 mg/kg Körpergewicht pro Tag. Die Dauer der Behandlung hängt von der Schwere der Erkrankung ab und kann von einer einmaligen Gabe bis zu einer mehrwöchigen oder sogar mehrmonatigen Behandlung, die gegebenenfalls in Intervallen wiederholt werden kann, reichen.

Die Erfindung soll durch die folgenden Beispiele und die beigefügten Figuren näher erläutert werden.

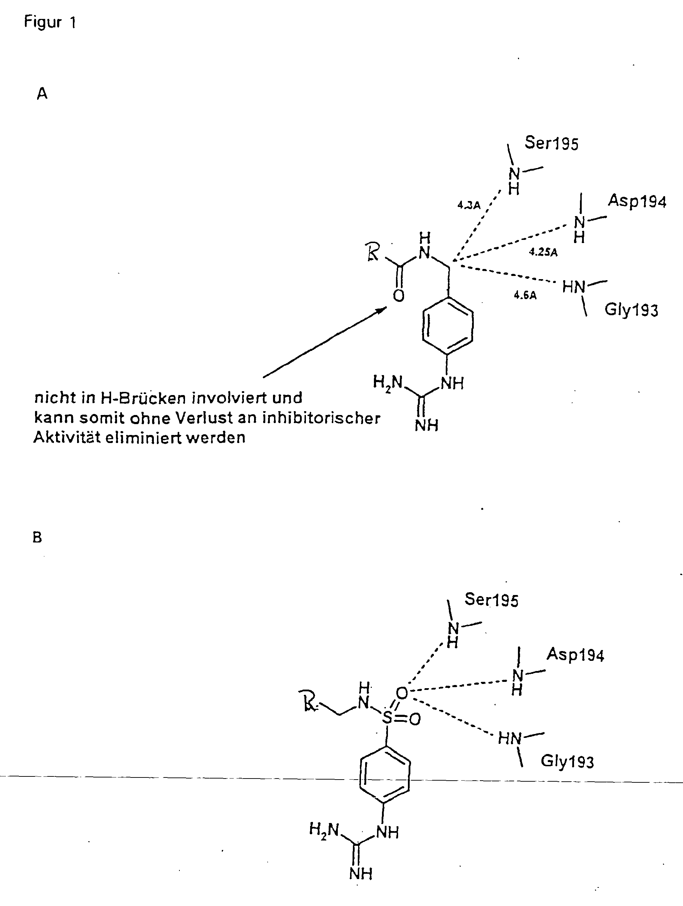

**Figur 1** zeigt die Wechselwirkungen zwischen erfindungsgemäßen Inhibitoren, in denen B eine SO₂-Gruppe darstellt mit Urokinase. Zu sehen sind die ausbildeten Wasserstoffbrücken zwischen der SO₂-Gruppe und den NH-Gruppen im Grundgerüst der Urokinase von Gly 193, Asp 194 und Ser 195.

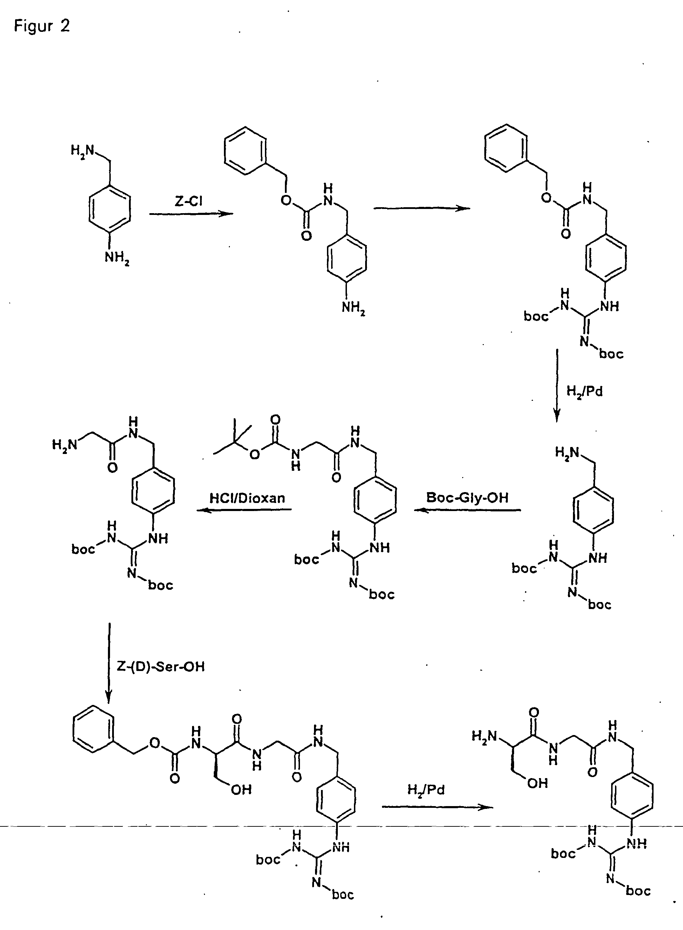

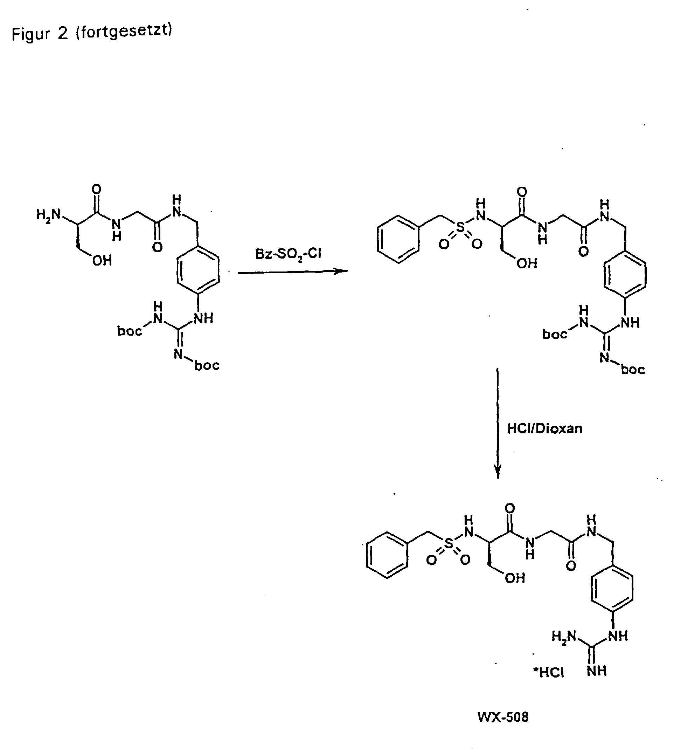

**Figur 2** zeigt ein Syntheseschema zur Herstellung der besonders bevorzugten Verbindung N-[(4-Guanidino-benzylcarbamoyl)-methyl]-3-hydroxy-2-phenylmethansulfonylaminopropionamid (WX-508). Die Verbindungen der Formel (I) können gemäß diesem allgemeinen Reaktionsschema, ausgehend von p-Amino-benzylamin, hergestellt werden. In Figur 2 bedeutet Z die Schutzgruppe Benzyloxycarbonyl und Boc die Schutzgruppe tert-Butyloxycarbonyl.

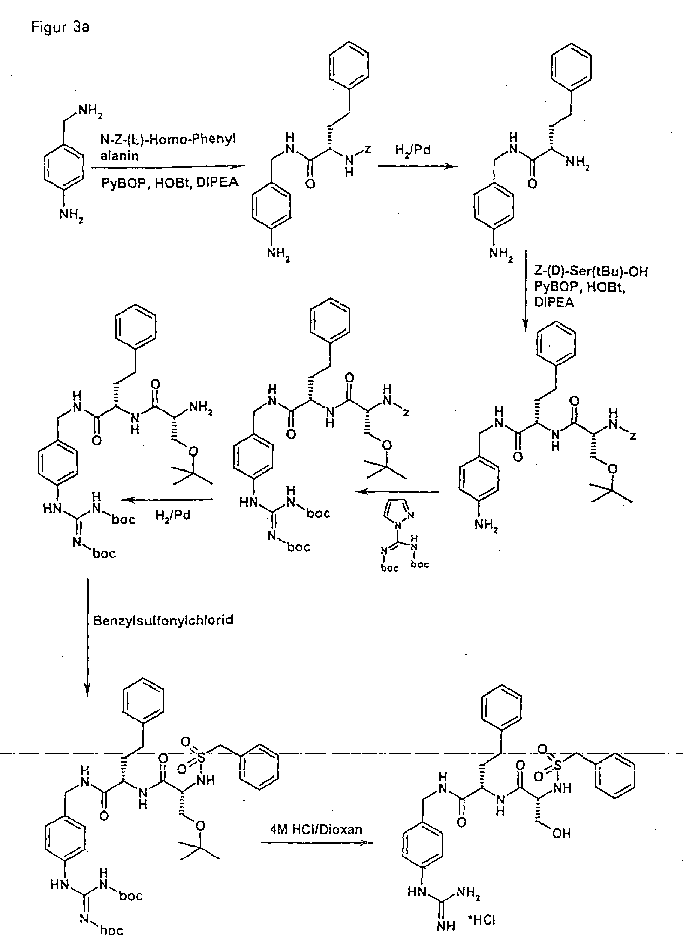

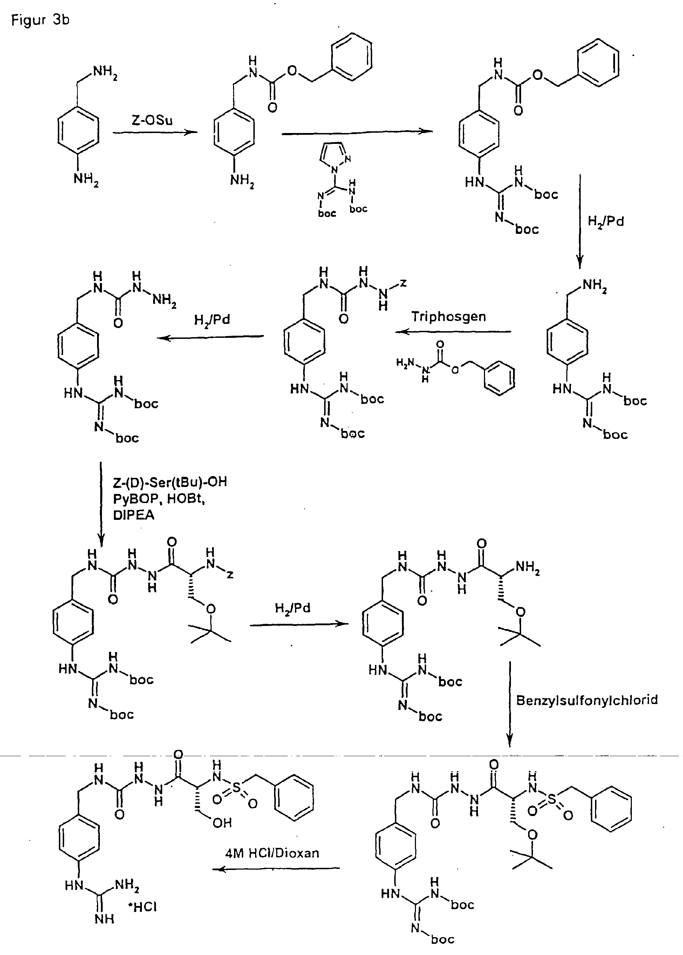

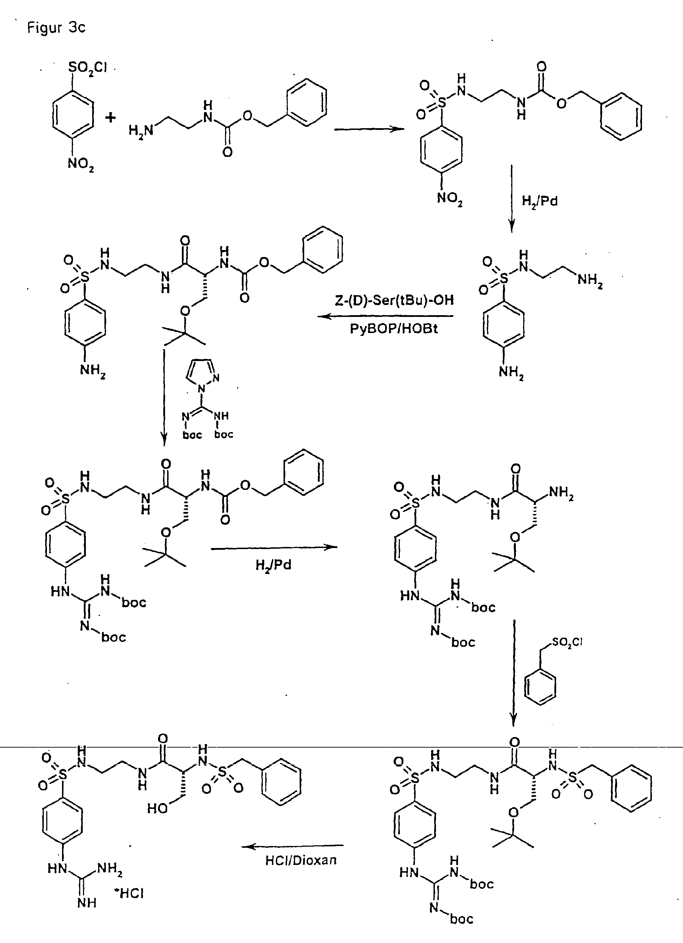

**Figur 3** zeigt Syntheseschemata zur Herstellung der besonders bevorzugten Verbindungen WX-550 (Figur 3a), WX-544 (Figur 3b) und WX-568 (Figur 3c). Die Verbindungen der Formel (I) können gemäß diesem allgemeinen Reaktionsschema, ausgehend von p-Amino-benzylamin, hergestellt werden. In Figur 3 bedeutet Z die Schutzgruppe Benzyloxycarbonyl und Boc die Schutzgruppe tert-Butyloxycarbonyl.

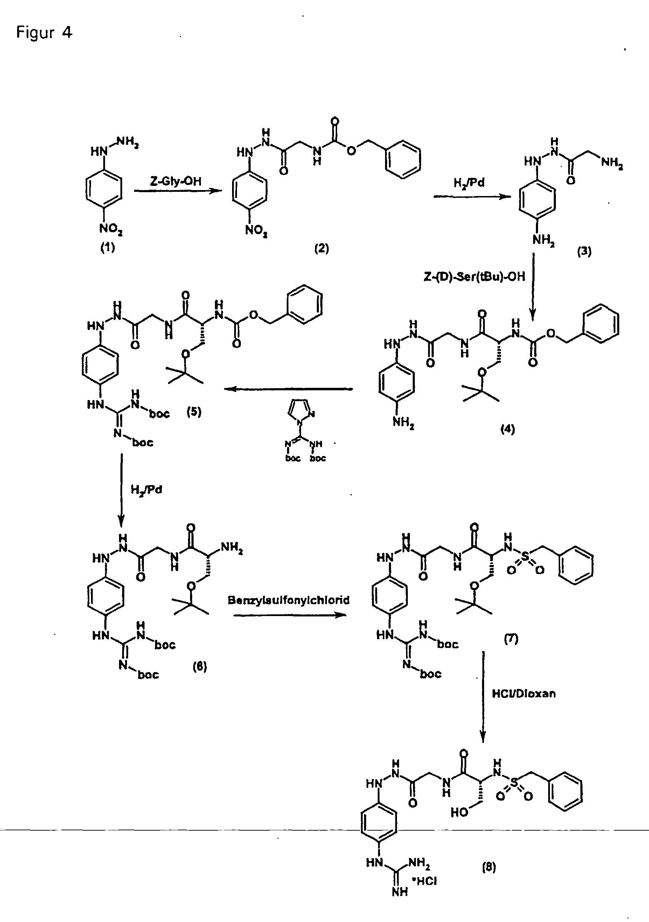

**Figur 4** zeigt ein Syntheseschema zur Herstellung der Verbindung WX-600.

### Beispiele

Hierin werden die folgenden Abkürzungen verwendet:

| | |
|---|---|
| HBTU | 2-(1H-Benzotriazol-1-yl)-1,1,3,3-tetramethyluroniumhexafluorophosphat |
| HOBt | N-Hydroxybenzotriazol |
| PyBOP | Benzotriazol-1-yl-oxy-tris- pyrrolidino-phosphoniumhexafluorophosphat |
| DCC | N,N'-Dicyclohexylcarbodiimid |
| tBu | tert-Butyl |
| BOC | tert-Butyloxycarbonyl-Schutzgruppe |
| Z | Benzyloxycarbonyl-Schutzgruppe |
| Z-OSu | N-(Benzyloxycarbonyloxy) succinimid |
| TEA | Triethylamin |
| DIPEA | Diisopropylethylamin |
| Z-Gly-OSu | Nα-Benzyloxycarbonylglycin-N-hydroxysuccinimidester |
| TFA | Trifluoressigsäure |
| 4M | 4 molar |
| N-Z-N-Me-Gly | Nα-Benzyloxycarbonyl--Nα-Methyl-Glycin |
| N-Z-N-Me-Ala | Nα-Benzyloxycarbonyl-Nα-methylalanin |
| Z-HomoPhe-OH | Nα-Benzyloxycarbonyl-Homophenylalanin |
| Fmoc-(D)Dap(Z)-OH | Nα-Fluorenyloxycarbonyl-N-Benzyloxycarbonyl-(D)-Diaminopropionsäure |
| BOC-(D)-Ser(tBu)-OH | Nα-tert-Butyloxycarbonyl-O-tert-butyl-(D)-Serin |

### Beispiel 1:

### Synthesebeschreibung N-[2-(4-Guanidino-benzenesulfonylamino)-ethyl]-3-hydroxy-2-phenylmethansulfonylamino-propionamide Hydrochloride [WX-568] (vgl. Fig. 3c)

4-Nitrophenylsulfonylchlorid (1) wird in einem inerten organischen Lösungsmittel (z. B. Dichlormethan) unter Zugabe einer organischen Base (z.B. TEA, DIPEA) mit N-Z-Diaminoethan Hydrochlorid (2) zur Verbindung 3 umgesetzt. Katalytische Hydrierung von 3 an einem Pd-Aktivkohle-Katalysator wandelt die Nitrogruppe zum korrespondierenden Amin um und spaltet zugleich die Z-Schutzgruppe ab (4). Die Aminoethylgruppe von Verbindung 4 lässt sich mit in der Peptidchemie üblichen Kupplungsreagenzien (z.B. PyBOP, HBTU oder DCC und HOBt) zusammen mit Z-(D)-Ser(tBu)-OH zum entsprechenden Amid 5 umsetzen. Die Umsetzung zum vollgeschützten Intermediat 6 erfolgt mit einem geeigneten Guanidinylierungsreagens wie z.B. N,N'-Bis(tert-butoxycarbonyl)-1H-pyrazol-1-carboxamidin. Anschließende Abspaltung der Z-Schutzgruppe durch katalytische Hydrierung am Pd-Aktivkohle-Katalysator (7) und Umsetzung mit Benzylsulfonylchlorid in einem inerten organischen Lösungsmittel (z.B. Dichlormethan) unter Zugabe einer organischen Base liefert das vollgeschützte Produkt 8. Die Abspaltung der Schutzgruppen (BOC und t-Butylether) erfolgt durch Lösen der Verbindung 8 in Säure (z.B. Trifluoressigsäure oder 4M HCl_{g} in Dioxan), wodurch das korrespondierende Salz der Zielverbindung N-[2-(4-Guanidinobenzenesulfonylamino)-ethyl]-3-hydroxy-2-phenylmethanesulfonylaminopropionamide (9) erhalten wird.

### Beispiel 2:

### Synthesebeschreibung Bz-SO₂-(D)-Ser-(Aza-Gly)-4-Guanidinobenzylamid Hydrochlorid [WX-544] (vgl. Fig. 3b)

Die Aminomethylgruppe des Edukts 4-Aminobenzylamin (10) wird zunächst mit einer geeigneten Schutzgruppe versehen, etwa durch Umsetzen von 10 mit Chlorameisensäurebenzylester oder Z-OSu zu Verbindung 11. Die Umsetzung mit einem geeignet geschützten Guanidinylierungsreagens wie z.B. N,N'-Bis(tert-butoxy-carbonyl)-1H-pyrazol-1-carboxamidin liefert Verbindung 12, deren Z-Schutzgruppe durch katalytische Hydrierung an einem Pd-Aktivkohle-Katalysator abgespalten werden kann (13). Die somit freigesetzte Aminofunktion wird unter Kühlung und Zugabe von einem Äquivalent organischer Base mit Triphosgen (einem festen und somit weniger giftigen Phosgenersatz) und anschließend in situ mit Benzylcarbazat zu Verbindung 14 [Z-AzaGly-4-(N,N'-Bis-BOC-Guanidinobenzyl)amid] umgesetzt. Die Z-Schutzgruppe wird wie für Verbindung 13 beschrieben durch Hydrieren abgespalten (15) und mit Z-(D)-Ser(tBu)-OH und in der Peptidchemie üblichen Kupplungsreagenzien (z.B. PyBOP, HBTU oder DCC und HOBt) zum Amid 16 umgesetzt. Die Z-Schutzgruppe wird erneut katalytisch abgespalten (17) und das resultierende freie Amin mit Benzylsulfonylchlorid in einem inerten organischen Lösungsmittel (z.B. Dichlormethan) unter Zugabe einer organischen Base zum korrespondierenden Sulfonamid 18 umgesetzt. Die Abspaltung der verbliebenen Schutzgruppen erfolgt durch Reaktion in saurer Lösung (z.B. in Trifluoressigsäure oder in 4M HCl_{g}/Dioxan) zum entsprechenden Salz der Zielverbindung Bz-SO₂-(D)-Ser-(Aza-Gly)-4-Guanidinobenzylamid (19).

### Beispiel 3:

### Synthesebeschreibung N-(4-Guanidino-benzyl)-2-(3-hydroxy-2-phenylmethane-sulfonylamino-propionylamino)-4-phenyl-butyramide Hydrochlorid [WX-550] (vgl. Fig. 3a)

4-Aminobenzylamin (20) wird mit Z-(L)_Homophenylalanin und in der Peptidchemice üblichen Kupplungsreagenzien (z.B. PyBOP, HBTUoder DCC und HOBt) zum Amid 21 umgesetzt. Durch katalytische Hydrierung an einem Pd-Aktivkohle-Katalysator wird die Z-Schutzgruppe abgespalten (22) und die freie Aminogruppe wie für 21 beschrieben mit Z-(D)-Ser(tBu)_OH zum Amid 23 umgesetzt. Die Umsetzung mit einem geeignet geschützten Guanidinylierungsreagens wie z.B. N,N'-Bis(tert-butoxycarbonyl)-1H-pyrazol-1-carboxamidin liefert Verbindung 24. Die N-terminale Z-Schutzgruppe wird wie für 22 beschrieben abgespalten und die resultierende Verbindung 25 mit Benzylsulfonylchlorid zum Sulfonamid 26 umgesetzt. Im letzten Schritt werden die verbliebenen Schutzgruppen in saurem Medium (z.B. in Trifluoressigsäure oder in 4M HCl_{g}/Dioxan) zum entsprechenden Salz der Zielverbindung 27 abgespalten.

### Beispiel 4:

### Synthese von N-[(4-Guanidino-benzylcarbamoyl)-methyl]-3-hydroxy-2-phenyl-methansulfonylamino-propionamid Hydrochlorid bzw. Benzylsulfonyl-(D)-Ser-Gly-(4-Guanidinobenzyl)amid Hydrochlorid (WX-508) (vgl. Fig. 2)

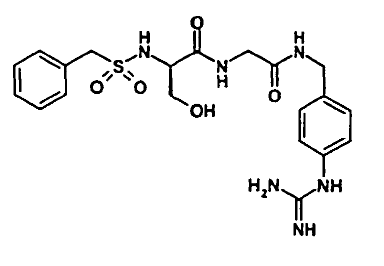

Kommerziell erhältliches 4-Aminobenzylamin wird in einem inerten Lösungsmittel mit Z-Gly-OSu zu Z-Gly-(4-Aminobenzyl)amid umgesetzt. (Alternativ können auch Z-Gly-OH und in der Peptidchemie übliche Kupplungsreagenzien (z.B. DCC oder HBTU und HOBt) verwendet werden. Nach Abspalten der Z-Schutzgruppe durch katalytische Hydrierung an einem Palladium/Aktivkohle-Katalysator, wird die freie Aminogruppe mit Hilfe von in der Peptidchemie üblichen Kupplungsreagenzien (z.B. DCC oder HBTU oder HOBt) mit Z-(D)-Ser(tBu)-OH zum entsprechenden Z-(D)-Ser(tBu)-Gly-(4-Aminobenzyl)amid umgesetzt. Der Aufbau der BOC-geschützten Guanidinofunktion kann z.B. auf dieser Stufe durch Reaktion mit N,N'-Bis-BOC-1 H-pyrazol-1-carboxamidin in einem möglichst unpolaren und inerten Lösungsmittel (z.B. Dichlormethan) erfolgen. Nach erneuter katalytischer Abspaltung der Z-Schutzgruppe erfolgt die Umsetzung mit Phenylmethansulfonylchlorid zum vollgeschützten Produkt BzSO₂-(D)-Ser(tBu)-Gly-(4-N,N'-Bis-BOC-Guanidinobenzyl)amid. Im letzten Syntheseschritt werden die BOC-Schutzgruppen und die tert-Butylethergruppe durch 4M HCl_{g} in Dioxan abgespalten, wodurch direkt das Hydrochlorid von N-[(4-Guanidino-benzylcarbamoyl)-methyl]-3-hydroxy-2-phenyl-methansulfonylamino-propionamid erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### Beispiel 5:

### Synthese des gemeinsamen Buildingblocks H-(D)-Ser(tBu)-Gly-(4,N,N'-Bis-BOC-Guanidino-Benzyl)amid (1):

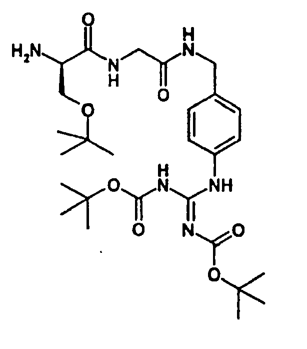

Kommerziell erhältliches 4-Aminobenzylamin wird in einem inerten Lösungsmittel mit Z-Gly-OSu zu Z-Gly-(4-Aminobenzyl)amid umgesetzt. [Alternativ können auch Z-Gly-OH und in der Peptidchemie übliche Kupplungsreagenzien (z.B. DCC oder HBTU und HOBt) verwendet werden]. Der Aufbau der BOC-geschützten Guanidinofunktion kann z.B. auf dieser Stufe durch Reaktion mit N,N'-Bis-BOC-1H-pyrazol-1-carboxamidin in einem möglichst unpolaren und inerten Lösungsmittel (z.B. Dichlormethan) erfolgen. Nach Abspalten der Z-Schutzgruppe durch katalytische Hydrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Z-(D)-Ser-(tBu)-OH zum entsprechenden Z-(D)-Ser(tBu)-Gly-(4-N,N'-Bis-BOC-Guandinobenzyl)amid umgesetzt. Abspaltung der Z-Schutzgruppe durch katalytische Hydrierung an einem Pd-Aktivkohlekatalysator liefert H-(D)-Ser(tBu)-Gly-(4-N,N'-Bis-BOC-Guanidinobenzyl)amid als Buildingblock (1) für die im Folgenden beschriebenen N-terminalen Derivatisierungsreaktionen.

### Beispiel 6:

### Synthese von N-terminalen Sulfonamidderivaten der allgemeinen Formel

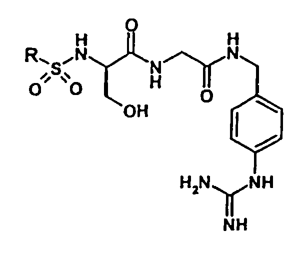

### (Beispiele WXC-296, 298, 300, 302, 316, 318, 340)

Zur Synthese der erfindungsgemäßen N-terminalen Sulfonamidderivate wird der Buildingblock 1 mit einem Äquivalent des gewünschten substituierten Sulfonylchlorids in Gegenwart einer tertiären organischen Base (z.B. TEA oder DIPEA) in einem inerten organischen Lösungsmittel (z.B. Dichlormethan) umgesetzt. Anschließend werden die Schutzgruppen des dabei gebildeten vollgeschützten Produkts durch 4M HCL_{g} in Dioxan abgespalten, wodurch das Hydrochlorid der gewünschten Verbindungen R-SO₂-(D)-Ser-Gly-(4-Guanidinobenzyl)amid erhalten wird (R steht allgemein für einen organischen Rest). Alternativ können die Schutzgruppen auch durch TFA abgespalten werden. Das entstandene TFA-Salz des Produkts wird anschließend durch Ionenaustausch in das korrespondierende Hydrochlorid überführt.

### 6.1. Vergleichsbeispiel: (-)-Camphor-10-sulfonyl-(D)-Ser-Gly-(4-Guanidinobenzyl)amid Hydrochlorid (WXC-296)

Zur Synthese der erfindungsgemäßen Verbindung (-)-Camphor-10-sulfonyl-(D)-Ser-Gly-(4-Guanidinobenzyl)amid Hydrochloird (WXC-296) wird der Buildingblock 1 mit einem Äquivalent (-)-Camphor-10-sulfonylchlorid in Gegenwart einer tertiären organischen Base (z.B. TEA oder DIPEA) in einem inerten organischen Lösungsmittel (z.B. Dichlormethan) umgesetzt.

Anschließend werden die Schutzgruppen des dabei gebildeten vollgeschützten Produkts durch 4M HCl_{g} in Dioxan abgespalten, wodurch das Hydrochlorid der Zielverbindung erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### 6.2. Vergleichsbeispiel: (+)-Camphor-10-sulfonyl-(D)-Ser-Gly-(4-Guanidinobenzyl)amid Hydrochlorid (WXC-298)

Die Synthese von (+)-Camphor-10-sulfonyl-(D)-Ser-Gly-(4-Guanidinobenzyl)amid Hydrochlorid (WXC-298) verläuft wie unter 6.1. beschrieben allerdings mit (+)-Camphor-10-sulfonylchlorid.

### 6.3. Vergleichsbeispiel: n-Butyl-sulfonyl-(D)-Ser-Gly-(4-Guanidinobenzyl)amid Hydrochlorid (WXC-300)

Die Synthese von n-Butyl-sulfonyl-(D)-Ser-Gly-(4-Guanidinobenzyl)amid Hydrochlorid (WXC-300) verläuft wie unter 6.1. beschrieben allerdings mit -Butylsulfonylchlorid.

### 6.4. Vergleichsbeispiel: n-Octyl-sulfonyl-(D)-Ser-Gly-(4-Guanidinobenzyl)amid Hydrochlorid (WXC-302)

Die Synthese von n-Octyl-sulfonyl-(D)-Ser-Gly-(4-Guanidinobenzyl)amid Hydrochlorid (WXC-302) verläuft wie unter 6.1. beschrieben allerdings mit n-Octylsulfonylchlorid.

### 6.5. 3-Nitrobenzyl-sulfonyl-(D)-Ser-Gly-(4-Guanidinobenzyl)amid Hydrochlorid (WXC-316)

Die Synthese von 3-Nitrobenzyl-sulfonyl-(D)-Ser-Gly-(4-Guanidinobenzyl)amid Hydrochlorid (WXC-316) verläuft wie unter 6.1. beschrieben allerdings mit 3-Nitrobenzyl-sulfonylchlorid.

### 6.6. 3-Chlorbenzyl-sulfonyl-(D)-Ser-Gly-(4-Guanidinobenzyl)amid Hyrochlorid (WXC-318)

Die Synthese von 3-Chlorbenzyl-sulfonyl-(D)-Ser-Gly-(4-Guanidinobenzyl)amid Hydrochlorid (WXC-318) verläuft wie unter 6.1 beschrieben allerdings mit 3-Chlorbenzyl-sulfonylchlorid.

### 6.7. 4-Chlorbenzyl-sulfonyl-(D)-Ser-Gly-(4-Guanidinobenzyl)amid Hydrochlorid (WXC-340)

Die Synthese von 4-Chlorbenzyl-sulfonyl-(D)-Ser-Gly-(4-Guanidinobenzyl)amid Hydrochlorid (WXC-340) verläuft wie unter 6.1. beschrieben allerdings mit 4-Chlorbenzyl-sulfonylchlorid.

### Beispiel 7:

### Synthese von N-terminalen Harnstoffderivaten der allgemeinen Formel

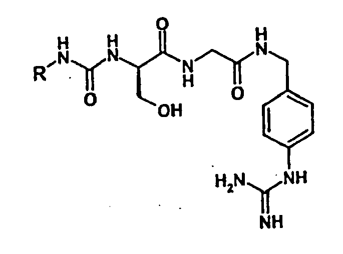

### (Vergleichsbeispiele: WXC-202, 304, 306, 308, 310, 312, 314, 320, 322, 324, 326, 328, 330, 332, 334, 336, 338, 342)

Zur Synthese der erfindungsgemäßen N-terminalen Harnstoffderivate wird der Buildingblock 1 mit einem Äquivalent des gewünschten substituierten Isocyanats in einem inerten organischen Lösungsmittel (z.B. Dichlormethan) umgesetzt. Anschließend werden die Schutzgruppen des dabei gebildeten vollgeschützten Produkts durch 4M HCl_{g} in Dioxan abgespalten, wodurch das Hydrochlorid der gewünschten Verbindungen N-[(4-Guanidinobenzylcarbamoyl)methyl]-3-hydroxy-2-(3-R-ureido)-propionamid erhalten wird (R stellt allgemein für einen organischen Rest). Alternativ können die Schutzgruppen auch durch TFA abgespalten werden. Das entstandene TFA-Salz des Produkts wird anschließend durch Ionenaustausch in das korrespondierende Hydrochlorid überführt.

### 7.1. Vergleichsbeispiel: 3-Chlorphenyl-aminocarbonyl-(D)-Ser-Gly-(4-Guanidinobenzyl)amid Hydrochlorid (WXC-292)

Zur Synthese der erfindungsgemäßen Verbindung 3-Chlorphenylaminocarbonyl-(D)-Ser-Gly-(4-Guanidinobenzyl)amid Hydrochlorid (WXC-292) wird der Buildingblock 1 mit einem Äquivalent 3-Chlorphenylisocyanat in einem inerten organischen Lösungsmittel (z.B. Dichlormethan) umgesetzt. Anschließend werden die Schutzgruppen des dabei gebildeten vollgeschützten Produkts durch 4M HCl_{g} in Dioxan abgespalten, wodurch das Hydrochlorid des gewünschten Produkts erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### 7.2. Vergleichsbeispiel: 2-Chlorphenyl-aminocarbonyl-(D)-Ser-Gly-(4-Guanidinobenzyl)amid Hydrochlorid (WXC-304)

Die Synthese von 2-Chlorphenyl-aminocarbonyl-(D)-Ser-Gly-(4-Guanidinobenzyl)amid Hydrochlorid (WXC-304) verläuft wie unter 7.1. beschrieben allerdings mit 2-Chlorphenylisocyanat.

### 7.3. Vergleichsbeispiel: 4-Methoxyphenyl-aminocarbonyl-(D)-Ser-Gly-(4-Guanidinobenzyl)amid Hydrochlorid (WXC-306)

Die Synthese von 4-Methoxyphenyl-aminocarbonyl-(D)-Ser-Gly-(4-Guanidinobenzyl)amid Hydrochlorid (WXC-306) verläuft wie unter 7.1. beschrieben allerdings mit 4-Methoxyphenylisocyanat.

### 7.4. Vergleichsbeispiel: 3,4,5-Tri-Methoxyphenyl-aminocarbonyl-(D)-Ser-Gly-(4-Guanidinobenzyl)amid Hydrochlorid (WXC-308)

Die Synthese von 3,4,5-Tri-Methoxyphenyl-aminocarbonyl-(D)-Ser-Gly-(4-Guanidinobenzyl)amid Hydrochlorid (WXC-308) verläuft wie unter 7.1. beschrieben allerdings mit 3,4,5-Tri-Methoxyphenylisocyanat.

### 7.5. Vergleichsbeispiel: 4-Phenoxyphenyl-aminocarbonyl-(D)-Ser-Gly-(4-Guanidinobenzyl)amid Hydrochlorid (WXC-310)

Die Synthese von 4-Phenoxyphenyl-aminocarbonyl-(D)-Ser-Gly-(4-Guanidinobenzyl)amid Hydrochlorid (WXC-310) verläuft wie unter 7.1. beschrieben allerdings mit 4-Phenoxyphenylisocyanat.

### 7.6. Vergleichsbeispiel: 3-Ethoxycarbonylphenyl-aminocarbonyl-(D)-Ser-Gly-(4-Guanidinobenzyl)amid Hydrochlorid (WXC-312)

Die Synthese von 3-Ethoxycarbonylphenyl-aminocarbonyl-(D)-Ser-Gly-(4-Guanidinobenzyl)amid Hydrochlorid (WXC-312) verläuft wie unter 7.1. beschrieben allerdings mit Ethyl-3-Isocyanatobenzoat.

### 7.7. Vergleichsbeispiel: 3-Acetylphenyl-aminocarbonyl-(D)-Ser-Gly-(4-Guanidinobenzyl)amid Hydrochlorid (WXC-314)

Die Synthese von 3-Acetylphenyl-aminocarbonyl-(D)-Ser-Gly-(4-Guanidinobenzyl)amid Hydrochlorid (WXC-314) verläuft wie unter 7.1. beschrieben allerdings mit 3-Acetylphenylsiocyanat.

### 7.8. Vergleichsbeispiel: 1-Adamantyl-aminocarbonyl-(D)-Ser-Gly-(4-Guanidinobenzyl)amid Hydrochlorid (WXC-320)

Die Synthese von 1-Adamantyl-aminocarbonyl-(D)-Ser-Gly-(4-Guanidiriobenzyl)amid Hydrochlorid (WXC-320) verläuft wie unter 7.1. beschrieben allerdings mit 1-Adamantylisocyanat.

### 7.9. Vergleichsbeispiel: 2-Bromphenyl-aminocarbonyl-(D)-Ser-Gly-(4-Guanidinobenzyl)amid Hydrochlorid (WXC-322)

Die Synthese von 2-Bromphenyl-aminocarbonyl-(D)-Ser-Gly-(4-Guanidinobenzyl)amid Hydrochlorid (WXC-322) verläuft wie unter 7.1. beschrieben allerdings mit 2-Bromphenylisdcyanat.

### 7.10. Vergleichsbeispiel: 3-Carboxyphenyl-aminocarbonyl-(D)-Ser-Gly-(4-Guanidinobenzyl)amid Hydrochlorid (WXC-324)

Die Synthese von 3-Carboxyphenyl-aminocarbonyl-(D)-Ser-Gly-(4-Guanidinobenzyl)amid Hydrochlorid (WXC-324) verläuft wie unter 7.1. beschrieben allerdings mit tert-Butyl-3-isocyanatobenzoate. Die tert-Butyl-Schutzgruppe wird gemeinsam mit den anderen säurelabilen Schutzgruppen durch Behandlung mit 4M HCl_{g} in Dioxan abgespalten.

### 7.11. Vergleichsbeispiel: 2,3-Dihydro-1,4-benzodioxin-6-yl-aminocarbonyl-(D)-Ser-Gly-(4-Guanidinobenzyl)amid Hydrochlorid (WXC-326)

Die Synthese von 2,3-Dihydro-1,4-benzodioxin-6-yl-aminocarbonyl-(D)-Ser-Gly-(4-Guanidinobenzyl)amid Hydrochlorid (WXC-326) verläuft wie unter 7.1. beschrieben allerdings mit 2,3-Dihydro-1,4-benzodioxin-6-yl-isocyanat.

### 7.12. Vergleichsbeispiel: 1-Naphthyl-aminocarbonyl-(D)-Ser-Gly-(4-Guanidinobenzyl)amid Hydrochlorid (WXC-328)

Die Synthese von 1-Naphthyl-aminocarbonyl-(D)-Ser-Gly-(4-Guanidinobenzyl)amid Hydrochlorid (WXC-328) verläuft wie unter 7.1. beschrieben allerdings mit 1-Naphthylisocyanat.

### 7.13. Vergleichsbeispiel: 4-Acetylphenyl-aminocarbonyl-(D)-Ser-Gly-(4-Guanidinobenzyl)amid Hydrochlorid (WXC-330)

Die Synthese von 4-Acetylphenyl-aminocarbonyl-(D)-Ser-Gly-(4-Guanidinobenzyl)amid Hydrochlorid (WXC-328) verläuft wie unter 7.1. beschrieben allerdings mit 4-Acetylphenylisocyanat.

### 7.14. Vergleichsbeispiel: 3,4-Methylendioxyphenyl-aminocarbonyl-(D)-Ser-Gly-(4-Guanidinobenzyl)amid Hydrochlorid (WXC-332)

Die Synthese von 3,4-Methylendioxyphenyl-aminocarbonyl-(D)-Ser-Gly-(4-Guanidinobenzyl)amid Hydrochlorid (WXC-332) verläuft wie unter 7.1. beschrieben allerdings mit 3,4-methylendioxyphenylisocyanat.

### 7.15. Vergleichsbeispiel: 2,3-Dichlorphenyl-aminocarbonyl-(D)-Ser-Gly-(4-Guanidinobenzyl)amid Hydrochlorid (WXC-334)

Die Synthese von 2,3-Dichlorphenyl-aminocarbonyl-(D)-Ser-Gly-(4-Guanidinobenzyl)amid Hydrochlorid (WXC-334) verläuft wie unter 7.1. beschrieben allerdings mit 2,3-Dichlorphenylisocyanat.

### 7.16.Vergleichsbeispiel:4-Ethyloxycarbonylphenyl-aminocarbonyl-(D)-Ser-Gly-(4-Guanidinobenzyl)amid Hydrochlorid (WXC-336)

Die Synthese von 4-Ethyloxycarbonylphenyl-aminocarbonyl-(D)-Ser-Gly-(4-Guanidinobenzyl)amid Hydrochlorid (WXC-336) verläuft wie unter 7.1. beschrieben allerdings mit 4-Ethyloxycarbonylphenylisocyanat.

### 7.17. Vergleichsbeispiel: 2,4-Dibromphenyl-aminocarbonyl-(D)-Ser-Gly-(4-Guanidinobenzyl)amid Hydrochlorid (WXC-338)

Die Synthese von 2,4-Dibromphenyl-aminocarbonyl-(D)-Ser-Gly-(4-Guanidinobenzyl)amid Hydrochlorid (WXC-338) verläuft wie unter 7.1. beschrieben allerdings mit 2,4-Dibromphenylsiocyanat.

### 7.18. Vergleichsbeispiel: 4-Nitrophenyl-aminocarbonyl-(D)-Ser-Gly-(4-Guanidinobenzyl)amid Hydrochlorid (WXC-342)

Die Synthese von 4-Nitrophenyl-aminocarbonyl-(D)-Ser-Gly-(4-Guanidinobenzyl)amid Hydrochlorid (WXC-342) verläuft wie unter 7.1. beschrieben allerdings mit 4-Nitrophenylisocyanat.

### Beispiel 8:

### Synthese von N-terminalen Amid-Derivaten der allgemeinen Formel

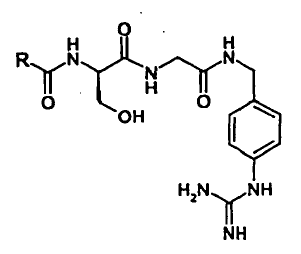

### (Beispiel WX-571)

Zur Synthese der erfindungsgemäßen N-terminalen Amidderivate wird der Buildingblock 1 mit einem Äquivalent des gewünschten substituierten Carbonsäurechlorids in Gegenwart einer tertiären organischen Base (z.B. TEA oder DIPEA) in einem inerten organischen Lösungsmittel (z.B. Dichlormethan) umgesetzt. Alternativ lassen sich auch Carbonsäuren mit in der Peptidchemie üblichen Kupplungsreagenzien (z.B. DCC oder HBTU oder HOBt) zum gewünschten Amid umsetzen. Andere Formen der Carbonsäureaktivierung z.B. als Pentafluorphenylester, Hydroxysuccinimidester oder als Anhydrid sind ebenso möglich. Anschließend werden die Schutzgruppen des dabei gebildeten vollgeschützten Produkts durch 4M HCl_{g} in Dioxan abgespalten, wodurch das Hydrochlorid der gewünschten Verbindungen N-[(4-Guanidinobenzylcarbamoyl)-methyl]-3-hydroxy-2-R-Acylamino-propionamid erhalten wird (R steht allgemein für einen organischen Rest). Alternativ können die Schutzgruppen auch durch TFA abgespalten werden. Das entstandene TFA-Salz des Produkts wird anschließend durch Ionenaustausch in das korrespondierende Hydrochlorid überführt.

### 8.1. Vergleichsbeispiel: 3,4-Dihydroxyphenyl-acetyl-(D)-Ser-Gly-(4-Guanidino-benzyl)amid Hydrochlorid (WX-571)

Zur Synthese der erfindungsgemäßen Verbindung 3,4-Dihydroxyphenylacetyl-(D)-Ser-Gly-(4-Guanidino-benzyl)amid Hydrochlorid (WX-571) wird der Buildingblock 1 mit einem Äquivalent 3,4-Dihydroxyphenylessigsäure und 1,1 Äquivalenten eines in der Peptidchemie üblichen Kupplungsreagenz (z.B. DCC oder HBTU und HOBt) in Gegenwart einer tertiären organischen Phsae (z.B. TEA oder DIPEA) in einem inerten organischen Lösungsmittel (z.B. Dichlormethan) umgesetzt. Anschließend werden die Schutzgruppen des dabei gebildeten vollgeschützten Produkts durch 4M HCl_{g} in Dioxan abgespalten, wodurch das Hydrochlorid der gewünschten Verbindungen gebildet wird. Alternativ können die Schutzgruppen auch durch TFA abgespalten werden. Das entstandene TFA-Salz des Produkts wird anschließend durch Ionenaustausch in das korrespondierende Hydrochlorid überführt.

### Beispiel 9:

### Synthese von BzSO₂-(D)-Ser-AzaGly-(4-Guanidinobenzyl)amid (WX-544)

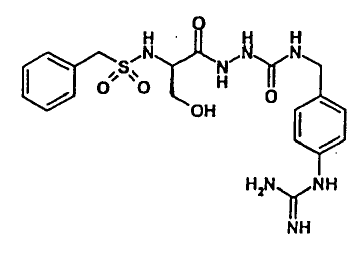

Zur Synthese der erfindungsgemäßen Verbindung BzSO₂-(D)-Ser-AzaGly-(4-Guanidinobenzyl)amid wird zunächst die Aminomethylgruppe von 4-Aminobenzylamin mit Z-OSu zu N-Z-(4-Aminobenzyl)amin umgesetzt. Der Aufbau der BOC-geschützten Guanidinofunktion kann z.B. auf dieser Stufe durch Reaktion mit N,N'-Bis-BOC-1H-pyrazol-1-carboxamidin in einem möglichst unpolaren und inerten Lösungsmittel (z.B. Dichlormethan) erfolgen. Nach Abspalten der Z-Schutzgruppe durch katalytische Hydrierung an einem Palladium/Aktivkohle-Katalysator, wird die freie Aminogruppe zunächst in einem inerten Lösungsmittel (z.B. Dichlormethan) unter Einskühlung mit 1/3 Äquivalent Triphosgen und einem Äquivalent TEA, und nach erfolgter Reaktion mit Benzylcarbazat zu Z-AzaGly-(4-N,N'-Bis-BOC-Guanidinobenzyl)amid umgesetzt. Nach Abspalten der Z-Schutzgruppe durch katalytische Hyrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Z-(D)-Ser(tBu)-OH zum entsprechenden Z-(D)-Ser(tBu)-AzaGly-(4-N,N'-Bis-BOC-Guanidinobenzyl)amid umgesetzt. Nach erneuter katalytischer Abspaltung der Z-Schutzgruppe erfolgt die Umsetzung mit Phenylmethansulfonylchlorid zum vollgeschützten Produkt BzSO₂-(D)-Ser(tBu)-AzaGly-4(N,N'-Bis-BOC-Guanidinobenzyl)amid. Im letzten Syntheseschritt werden die BOC-Schutzgruppen und die tert-Butylethergruppe durch 4M HCl_{g} in Dioxan abgespalten, wodurch direkt das Hydrochlorid von BzSO₂-(D)-Ser-AzaGly-4-(Guanidinobenzyl)amid erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### Beispiel 10:

### Synthese der erfindungsgemäßen Verbindungen des Typs BzSO₂-(D)-Ser-Aaa-(4-Guanidinobenzyl)amid (Aaa bedeutet Homophenylalanin)

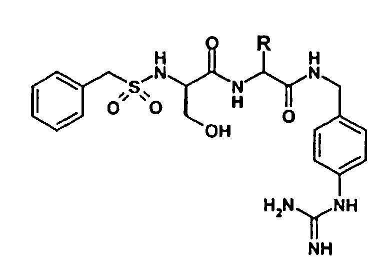

### (Beispiel WX-550)

Kommerziell erhältliches 4-Aminobenzylamin wird in einem inerten Lösungsmittel mit der gewünschten Z-geschützten Aminosäure (Z-Aaa) und in der Peptidchemie üblichen Kupplungsreagenzien (z.B. DCC oder HBTU und HOBt) zu Z-Aaa-(4-Aminobenzyl)amid umgesetzt. Evtl. vorhandene reaktive Gruppen in der Seitenkette der Aminosäure Aaa sollten dabei nach Möglichkeit mit einer säurelabilen Schutzgruppe (z.B. BOC, Trityl, tert-Butylester bzw. -ether) versehen sein. Nach Abspalten der Z-Schutzgruppe durch katalytische Hyrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Z-(D)-Ser(tBu)-OH zum entsprechenden Z-(D)-Ser(tBu)-Aaa-(4-Aminobenzyl)amid umgesetzt. Der Aufbau der BOC-geschützten Guanidinofunktion kann z.B. auf dieser Stufe durch Reaktion mit N,N'-Bis-BOC-1H-pyrazol-1-carboxamidin in einem möglichst unpolaren und inerten Lösungsmittel (z.B. Dichlormethan) erfolgen. Nach erneuter katalytischer Abspaltung der Z-Schutzgruppe erfolgt die Umsetzung mit Phenylmethansulfonylchlorid zum vollgeschützten Produkt BzSO₂-(D)-Ser(tBu)-Gly-4-(N,N'-Bis-BOC-Guanidinobenzyl)amid. Im letzten Syntheseschritt werden die BOC-Schutzgruppen und die tert-Buthylethergruppe durch 4M HCl_{g} in Dioxan abgespalten, wodurch direkt das Hydrochlorid von BzSO₂-(D)-Ser-Aaa-(4-Guanidinobenzyl)amid erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### 10.1 Benzylsulfonyl-(D)-Ser-HomoPhe-(4-Guanidinobenzyl)amid Hydrochlorid (WX-550)

Kommerziell erhältliches 4-Aminobenzylamin wird in einem inerten Lösungsmittel (z.B. Dichlormethan) mit Z-HomoPhe-OH und in der Peptidchemie üblichen Kupplungsreagenzien (z.B. DCC oder HBTU und HOBt) zu Z-HomoPhe-(4-Aminobenzyl)amid umgesetzt. Nach Abspalten der Z-Schutzgruppe durch katalytische Hydrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Z-(D)-Ser(tBu)-OH zum entsprechenden Z-(D)-Ser(tBu)-HomoPhe-(4-Aminobenzyl)amid umgesetzt. Der Aufbau der BOC-geschützten Guanidinofunktion kann z.B. auf dieser Stufe durch Reaktion mit N,N'-Bis-BOC-1H-pyrazol-1-carboxamidin in einem möglichst unpolaren und inerten Lösungsmittel (z.B. Dichlormethan) erfolgen. Nach erneuter katalytischer Abspaltung der Z-Schutzgruppe erfolgt die Umsetzung mit Phenylmethansulfonylchlorid zum vollgeschützten Produkt BzSO₂-(D)-Ser(tBu)-HomoPhe-4-(N,N'-Bis-BOC-Guanidino-benzyl)amid. Im letzten Syntheseschritt werden die BOC-Schutzgruppen und die tert-ButyletherGruppe durch 4M HCl_{g} in Dioxan abgespalten, wodurch direkt das Hydrochlorid von BzSO₂-(D)-Ser-HomoPhe-(4-Guanidinobenzyl)amid erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### Beispiel 11:

### 11.1 Synthese der erfindungsgemäßen Verbindung 2-(4-Chlorophenylmethansulfonylamino)-N-[1-(4-guanidino-benzylcarbamoyl)-ethyl]-3-hydroxy-N-methyl-propionamid bzw. 4-Chlorbenzylsulfonyl-(d)-Ser-N-Me-Ala-(4-Guaniinobenzylamid) (WX-582)

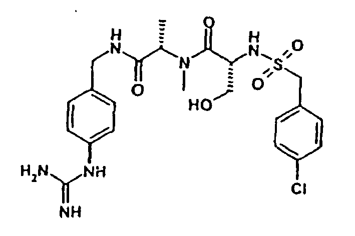

Zur Synthese der erfindungsgemäßen Verbindung wird N-Z-N-Methyl-Alanin mit in der Peptidchemie üblichen Kupplungsreagenzien (z.B. DCC oder HBTU oder HOBt) mit 4-Aminobenzylamin zu N-Z-N-Methyl-Alanin-(4-aminobenzyl)amid umgesetzt. Nach Abspalten der Z-Schutzgruppe durch katalytische Hydrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Z-(D)-Ser(tBu)-OH zum entsprechenden Z-(D)-Ser(tBu)-N-Methyl-Ala-(4-Aminobenzyl)amid umgesetzt. Der Aufbau der BOC-geschützten Guanidinofunktion kann z.B. auf dieser Stufe durch Reaktion mit N,N'-Bis-BOC-1H-pyrazol-1-carboxamidin in einem möglichst unpolaren und inerten Lösungsmittel (z.B. Dichlormethan) erfolgen. Nach erneuter katalytischer Abspaltung der Z-Schutzgruppe erfolgt die Umsetzung mit 4-Chlorophenylmethansulfonylchlorid zum vollgeschützten Produkt 4Cl-BzSO₂-(D)-Ser(tBu)-N-Methyl-Ala-(4-N,N'-Bis-BOC-Guanidinobenzyl)-amid. Im letzten Syntheseschritt werden die BOC-Schutzgruppen und die tert-Butylethergruppe durch 4M HCl_{g} in Dioxan abgespalten, wodurch direkt das Hydrochlorid von 2-(4-Chlorophenyl-methansulfonylamino)-N-[1-(4-guanidino-benzylcarbamoyl)-ethyl]-3-hydroxy-N-methyl-propionamid erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### 11.2 Synthese von Benzylsulfonyl-(D)-Ser-N-Me-Gly-(4-Guanidinobenzyl)amid (WX-538)

Die Synthese der erfindungsgemäßen Verbindung Benzylsulfonyl-(D)-Ser-N-Me-Gly-(4-Guanidinobenzyl)amid (WX-538) verläuft analog zu 11.1 allerdings mit N-Z-N-Me-Gly-OH anstelle von N-Z-N-Methyl-Alanin und mit Benzylsulfonylchlorid anstelle von 4-Chlorbenzylsulfonylchlorid.

### Beispiel 12 (Vergleichsbeispiel): Synthese von N-[N'-Guanidino-phenyl)-hydrazinocarbonylmethyl]-3-hydroxy-2-phenyl-methansulfonylaminopropionamid Hydrochlorid (WX-600) (vgl. Fig. 4)

4-Nitrophenylhydrazin (1) wird mit Z-Gly-OH und einem in der Peptidchemie üblichen Kupplungsreagenz (z.B. PyBOP, HBTU oder DCC und HOBt) in einem inerten organischen Lösungsmittel (z.B. Dichlormethan oder DMF) zu Verbindung 2 umgesetzt. Durch katalytische Hydrierung an einem Palladium-Aktivkohle-Katalysator lässt sich sowohl die Z-Schutzgruppe abspalten als auch die Nitrofunktion zur korrespondierenden Aminogruppe reduzieren, wodurch Aminoessigsäure-N'-(4-amino-phenyl)hydrazin (3) erhalten wird. Die aliphatische Aminofunktion lässt sich durch in der Peptidchemie übliche Kupplungsreagenzien (z.B. PyBOP, HBTU oder DCC und HOBt) in einem inerten organischen Lösungsmittel (z.B. Dichlormethan oder DMF) mit Z-(D)-Ser-(tBu)-OH zum mid 4 umsetzen. Der Aufbau der Bis-Boc-geschützten Guanidinofunktion (5) erfolgt durch Reaktion mit N,N'-Bis(tert-butoxycarbonyl)-1H-pyrazol-1-carboxamidin in einem inerten organischen (z.B. Dichlormethan). Durch erneute katalytische Hydrierung an einem Palladium-Aktivkohle-Katalysator wird die Z-Schutzgruppe abgespalten (6). Die anschließende Reaktion mit 1 Äquivalent Benzylsulfonylchlorid in Dichlormethan liefert das vollgeschützte Produkt 7, das durch Reaktion in 4M HCl_{g} in Dioxan zum Hydrochlorid des gewünschten Produkts N-[N'-(4-Guanidino-phenyl)-hydrazinocarbonylmethyl]-3-hydroxy-2-phenyl-methansulfonylaminopropionamid (8) entschützt wird.

### Beispiel 13:

### Synthese der Derivate mit der allgemeinen Formel

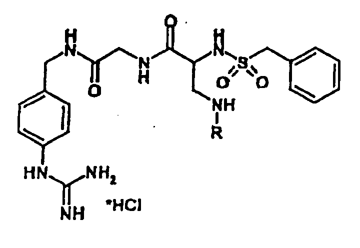

### 13.1. Vergleichsbeispiel: Synthese von Benzylsulfonyl-(D)-Dap(Z)-Gly-(4-Guanidino-benzyl)amid Hydrochlorid (WXM-5)

Kommerziell erhältliches 4-Aminobenyzlamin wird in einem inerten Lösungsmittel mit Z-Gly-OSu zu Z-Gly-(4-Aminobenzyl)amid umgesetzt. (Alternativ kann auch Z-Gly-OH und in der Peptidchemie übliche Kupplungsreagenzien (z.B. DCC oder HBTU und HOBt) verwendet werden. Nach Abspalten der Z-Schutzgruppe durch katalytische Hydrierung an einem Palladium/Aktivkohle-Katalysator, wird die freie Aminogruppe mit Hilfe von in der Peptidchemie üblichen Kupplungsreagenzien (z.B. DCC oder HBTU und HOBt) mit Fmoc-(D)-Dap(Z)-OH zum entsprechenden Fmoc-(D)-Dap(Z)-Gly-(4-Aminobenzyl)amid umgesetzt. Der Aufbau der BOC-geschützten Guanidinofunktion kann z.B. auf dieser Stufe durch Reaktion mit N,N'-Bis-BOC-1 H-pyrazol-1-carboxamidin in einem möglichst unpolaren und inerten Lösungsmittel (z.B. Dichlormethan) erfolgen. Nach Abspaltung der Fmoc-Schutzgruppe durch eine sekundäre organische Base (z.B. Diethylamin oder Piperidin) erfolgt die Umsetzung mit Phenylmethansulfonylchlorid zum geschützten Produkt BzSO₂-(D)-Dap(Z)-Gly-(4-N,N'-Bis-BOC-Guanidinobenzyl)amid. Im letzten Syntheseschritt werden die BOC-Schutzgruppen durch 4M HCl_{g} in Dioxan abgespalten, wodurch direkt das Hydrochlorid von Benzylsulfonyl-(D)-Dap(Z)-Gly-(4-Guanidino-benzyl)amid Hydrochlorid (WXM-5) erhalten wird. Alternativ können die Schutzgruppen auch durch TFA abgespalten werden. Das entstandene TFA-Salz des Produkts wird anschließend durch Ionenaustausch in das korrespondierende Hydrochlorid überführt.

### 13.2. Vergleichsbeispiel: Synthese von Benzylsulfonyl-(D)-Dap-Gly-(4-Guanidino-benzyl)amid Bis-Hydrochlorid (WXM-6)

Benzylsulfonyl-(D)-Dap-Gly-(4-Guanidino-benzyl)amid Bis-Hydrochlorid (WXM-6) kann durch katalytische Hydrierung von WXM-5 (siehe 13.1) an einem Palladium-Aktivkohle-Katalysator in Methanol das 2 molare Äquivalente 1 M HCl enthält, synthetisiert werden.

### Beispiel 14:

### In vitro Hemmung von Urokinase und Plasmin

Zur Bestimmung der Inhibitoraktivität wurden 200 µl Tris-Puffer (0,05 mol/l, den Inhibitor enthaltend, 0,154 mol/l NaCl, pH 8,0), 25 µl Substrat (Pefachrome uPA, Pefachrome PL oder Pefabloc TH in H₂O; Pentapharm LTD, Basel, Schweiz) und 50 µl Urokinase (Calbiochem-Novabiochem GmbH, Bad Soden, Deutschland) bzw. eine entsprechende andere Protease, z.B. Plasmin, Thrombin oder FXa, 10 Minuten bei 30 °C inkubiert. Nach ca. 30 Minuten und 30 Zyklen wird die Absorption bei 405 nm mittels eines Mikroplate Reader (Mediators PHL, Aureon Biosystems, Wien, Österreich) bestimmt. Die Kᵢ-Werte wurden nach Dixon durch lineare Regression mittels eines Computerprogramms ermittelt. Die Kᵢ-Werte sind das Mittel aus mindestens drei Bestimmungen, die Standardabweichung lag unter 25 %.

| **Formel** | Bez. | Ki [µM] Plasmin | Ki [µM] Thrombin | Ki [µM] FXa | Ki [µM] uPA |
|---|---|---|---|---|---|
| 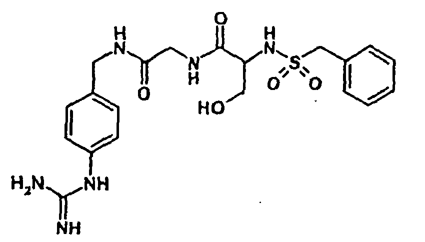 | WX-508 | >200 | > 350 | | 0,034 |
| 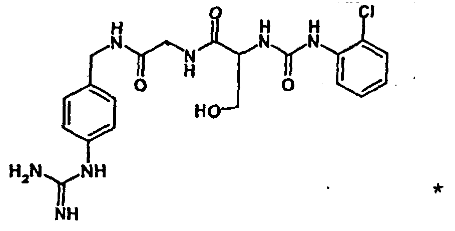 | WX-C304 | | | | no inh. *) |
| 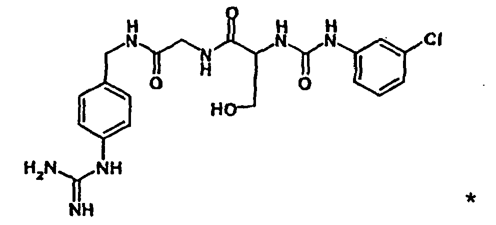 | WX-C292 | | | | no inh. *) |
| 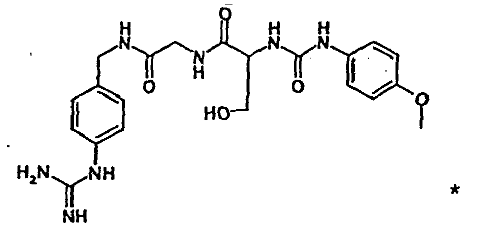 | WX-C 306 | | | | 4,1 |
| 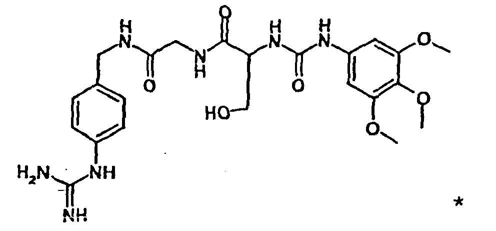 | WX-C 308 | | | | > 20 |
| 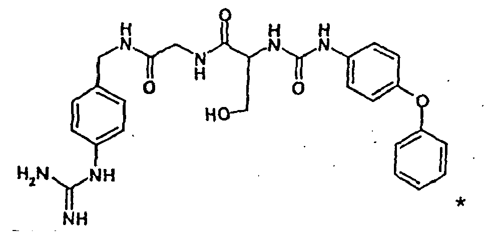 | WX-C 310 | | | | 2,0 |
| 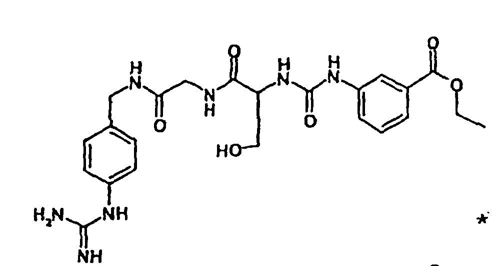 | WX-C 312 | | | | 1,4 |
| 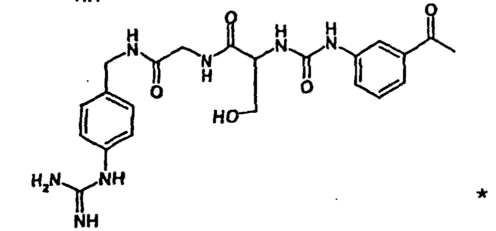 | WX-C 314 | | | | no inh. *) |
| 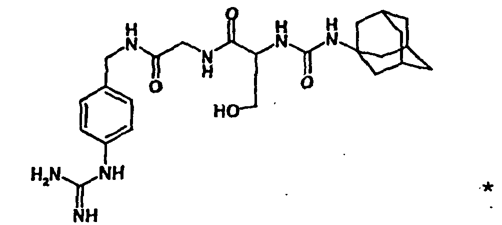 | WX-C 320 | | | | > 20 |
| 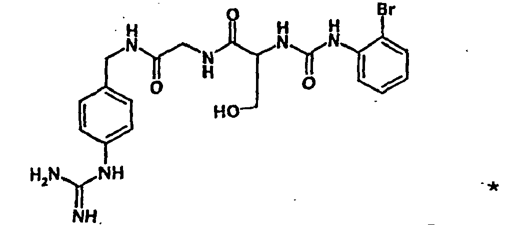 | WX-C 322 | | | | > 20 |
| 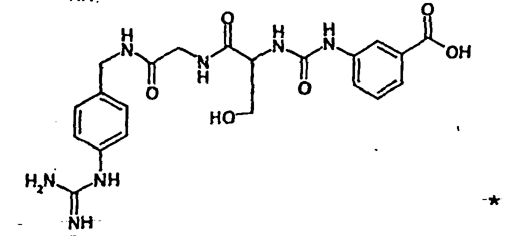 | WX-C 324 | | | | 3,1 |
| 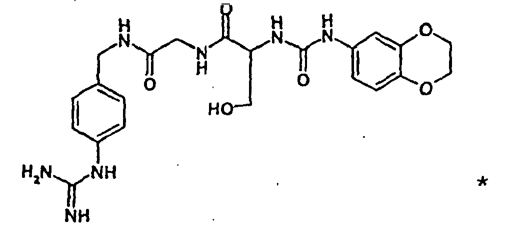 | WX-C 326 | | | | > 20 |
| 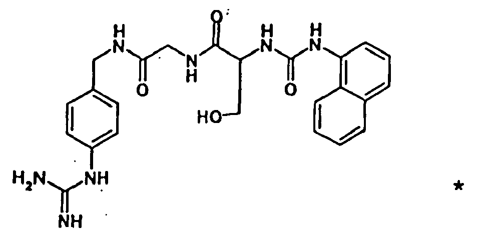 | WX-C 328 | | | | > 20 |
| 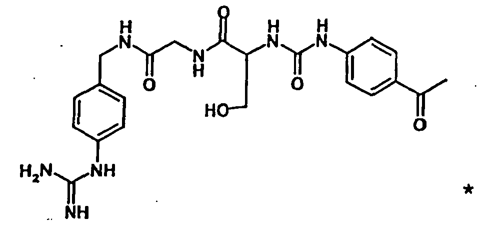 | WX-C 330 | | | | > 20 |
| 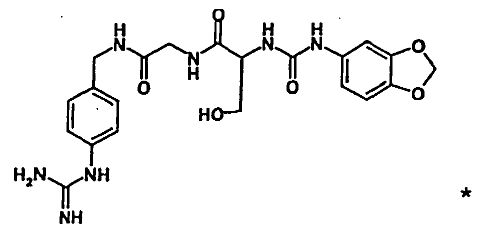 | WX-C 332 | | | | > 20 |
| 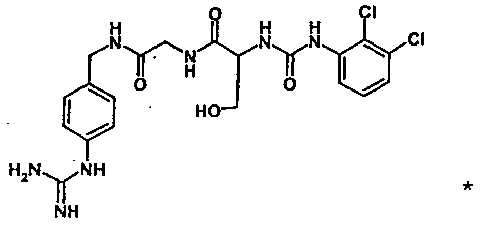 | WX-C 334 | | | | > 20 |
| 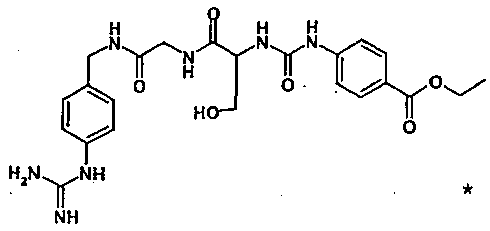 | WX-C 336 | | | | 13,1 |
| 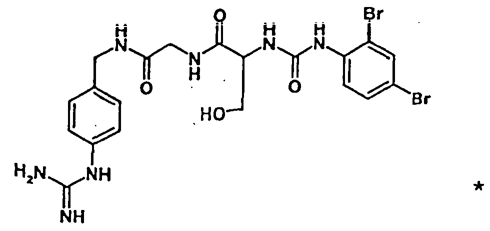 | WX-C 338 | | | | > 20 |
| 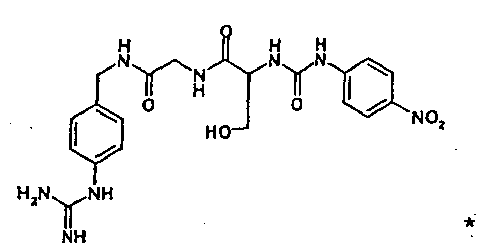 | WX-C342 | | | | 4,9 |
| 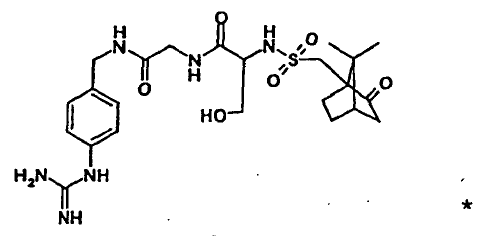 | WX-C 296 | | | | 0,45 |
| 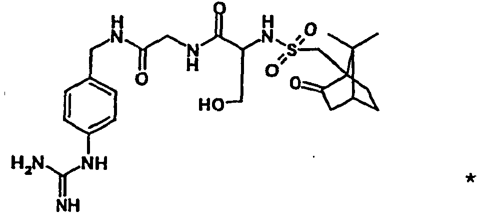 | WX-C 298 | | | | 0,16 |
| 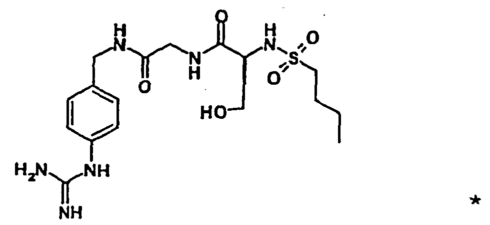 | WX-C 300 | | | | 0,27 |
| 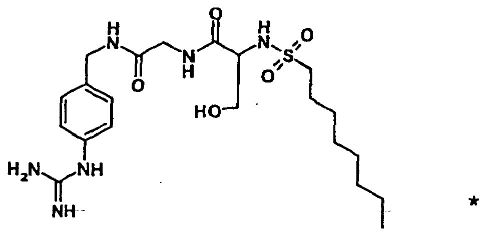 | WX-C 302 | | | | 0,5 |
| 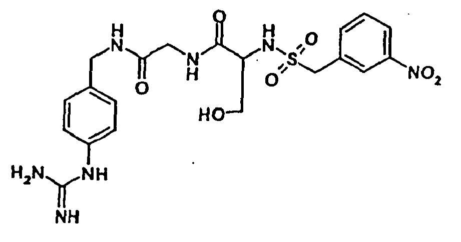 | WX-C 316 | no inh. at 100 µM *) | no inh. at 100 µM *) | 32,4 | 0,09 |
| 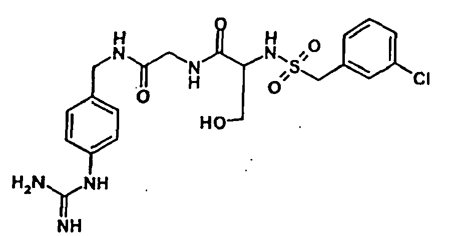 | WX-C 318 | no inh. at 100 µM *) | no inh. at 100 µM *) | 36 | 0,047 |
| 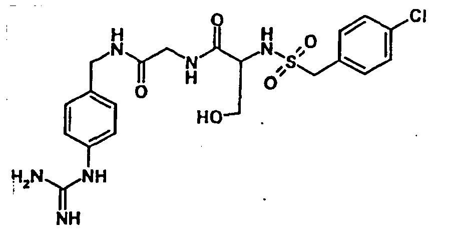 | WX-C 340 | no inh. at 100 µM *) | no inh. at 100 µM *) | 34 | 0,01 |
| 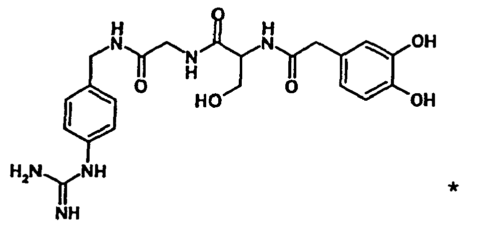 | WX- 571 | | | | 14,7 |
| 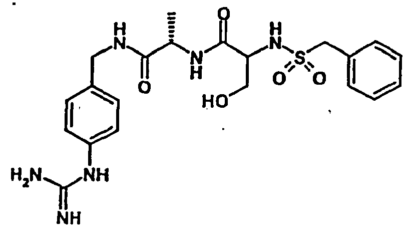 | WX- 532 | 40 | no inh. at 100 µM *) | | 0,016 |
| 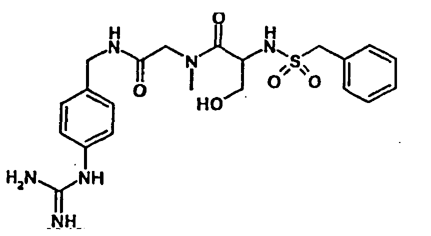 | WX- 538 | 130 | > 100 | > 100 | 0,041 |
| 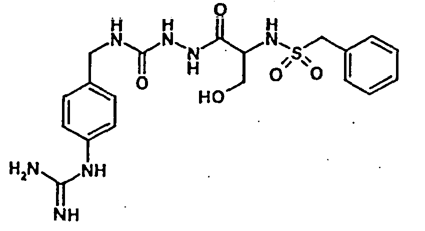 | WX- 544 | | no inh. at 100 µM *) | | 0,83 |
| 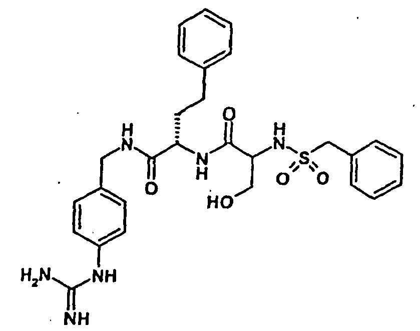 | WX- 550 | 2,5 | > 50 | > 100 | 0,10 |
| 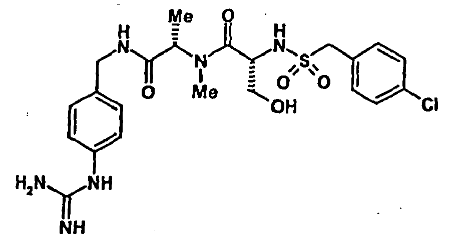 | WX- 582 | | | | 0,01 |
| 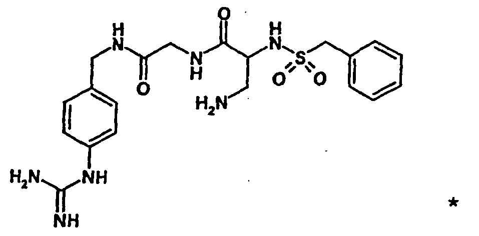 | WX- M6 | | | | 0,15 |
| 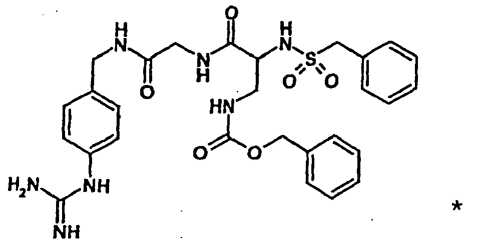 | WX- M5 | | | | 0,5 |
| 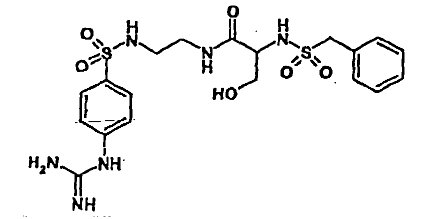 | WX- 568 | | > 1000 | > 1000 | 2,1 |
| 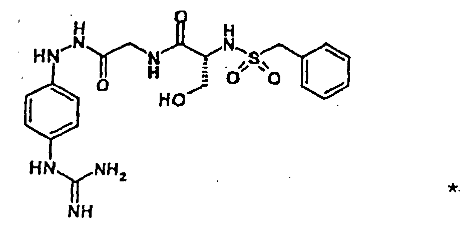 | WX- 600 | | | | 2,1 |

| | | | | | |
|---|---|---|---|---|---|
| * Vergleichsbeispiel *) no inh. = keine Inhibierung *) no inh. at = keine Inhibierung bei | | | | | |

### Beispiel 15

### In vitro Hemmung von Urokinase durch WX-508

Zur Bestimmung der uPA Inhibitoraktivität wurden 200 µl Tris-Puffer (0,05 mol/l, den Inhibitor enthaltend, 0,154 mol/l NaCl, pH 8,0), 25 µl Substrat (Pefachrome uPA oder BZ-β-Ala-Gly-Arg-pNA in H₂O; Pentapharm LTD, Basel, Schweiz) und 50 µl Urokinase (Calbiochem-Novabiochem GmbH, Bad Soden, Deutschland) bzw. eine entsprechende andere Protease bei 30 °C inkubiert. Nach ca. 30 Minuten und 30 Zyklen wird die Absorption bei 405 nm mittels eines Mikroplate Reader (Mediators PHL, Aureon Biosystems, Wien, Österreich) bestimmt. Die Kᵢ-Werte wurden nach Dixon durch lineare Regression mittels eines Computerprogramms ermittelt. Die Kᵢ-Werte sind das Mittel aus mindestens drei Bestimmungen, die Standardabweichung lag unter 25 %.

| | Kᵢ (µM) |
|---|---|
| Urokinase | 0,04 |
| Plasmin | > 1000 |
| Thrombin | > 1000 |

### Beispiel 16:

### Zellkulturexperimente (Caco-Assay)

Es wurde der Transport der Verbindungen WX-508, WXC-316, WXC-318, WXC-324, WXC-340, WX-532, WX-538, WX-550 und WX-582 über Caco-2-Zellmonoschichten untersucht. In diesem Versuch wurden die genannten Verbindungen hinsichtlich ihrer Bioverfügbarkeit nach oraler Applikation beurteilt.

### 16.1 Versuchsaufbau

### 16.1.1 Zellkultur:

Caco-2-Zellen (American Type Culture Collection, Rockville, MD, USA) werden in Dulbecco's Modified Eagle Medium (DMEM) (Life Technologies, Gibco BRL, UK) enthaltend 10 % Vol/Vol hitzedenaturiertes fötales Kälberserum (FCS), 1 % Vol/Vol nicht essenzielle Aminosäuren, 160 U/ml Benzylpenicillin und 100 U/ml Streptomycin (Sigma Chemical, St. Louis, MO, USA) kultiviert. Die Zellen werden bei 37 °C in einer Atmosphäre aus 95 % Luft und 5 % CO₂ bei 90 % relativer Luftfeuchtigkeit gehalten. Die Zellen werden in 25 cm3 Kulturkolben gezüchtet, wobei das Medium jeden zweiten Tag gewechselt wird und die Zellen einmal in der Woche trypsiniert werden.

### 16.1.2. Transportexperimente und transepithelialer elektrischer Widerstand (TEER)

Für Transportuntersuchungen werden die Caco-2-Zellen auf porösen Polycarbonatfiltermembranen mit einer Porengröße von 0,4 µm und einer Oberfläche von 4,7 cm² in Clustern von 6 Vertiefungen (Costar Transwell, Badhoevedorp, Niederlande) kultiviert. Die Zellen werden mit einer anfänglichen Dichte von 10⁴ Zellen/cm² auf jeden Filter geimpft. Diese Zellen werden bei 37 °C in einer wie oben beschriebenen Atmosphäre gehalten. Das Medium wird jeden zweiten Tag über drei Wochen ersetzt.

Am Tag der Transportexperimente wird das Kulturmedium mit dem gleichen Volumen an Hank's ausgeglichener Salzlösung (HBSS) gepuffert mit 30 mM HEPES bei pH 7,2 (Transportmedium) ersetzt und die Zellen werden für eine Stunde äquilibrieren gelassen. Danach wird das Apicalmedium durch 1,5 ml einer die Verbindungen enthaltende Lösung in HBSS/HEPES (10 µg/ml) ersetzt. Die Zellmonoschichten werden für vier Stunden inkubiert. Proben von den apicalen und basolateralen Kompartementen werden am Ende der Experimente genommen und zur quantitativen Bestimmung des Transports der jeweiligen Verbindungen herangezogen.

Der transepitheliale elektrische Widerstand (TEER) wird vor und nach dem Äquilibrieren unter Verwendung eines Milicell ERS-Meters gemessen, welches mit einem Paar Elektroden verbunden war, um die Integrität der auf den Filtern gebildeten Monoschichten sicherzustellen. TEER wird auch am Ende der Experimente gemessen, um sicherzustellen, dass die Zellmonoschicht zusammenhängend bleibt.

Permeabilitätsuntersuchungen unter Verwendung von ¹⁴C-Mannitol über Caco-2-Zellmonoschichten werden durch Zugabe von 1,5 ml der radioaktiv markierten ¹⁴C-Mannitollösung in HBSS-HEPES (4 mmol/l mit einer spezifischen Aktivität von 0,2 µCi/ml) zu der apicalen Seite durchgeführt. Proben von der apicalen und der basolateralen Seite werden entnommen und nach Zugabe eines Szintillationscocktails in einem Beta-Zähler analysiert. Alle Experimente wurden dreifach durchgeführt.

Das Flussschema des Experiments ist wie folgt:

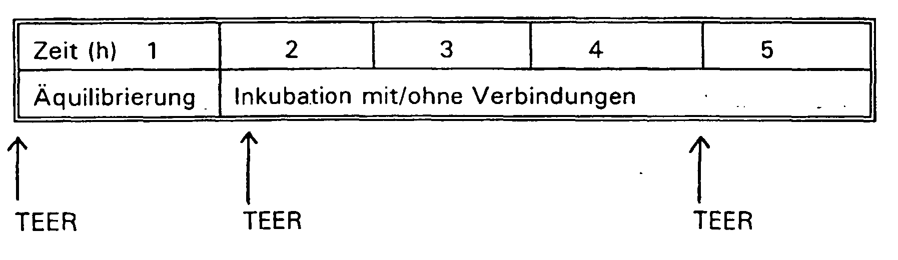

### Ergebnisse:

| Verbindung | % Menge im basolateralen Kompartement | | |
|---|---|---|---|
| WX-508 | 0,2 | 0,1 | 0,7 |
| WXC-316 | 0,2 | 0,2 | 3,6 |
| WXC-318 | 0,4 | 1,0 | 4,5 |
| WXC-324 * | 0,1 | 0,1 | 2,0 |
| WXC-340 | 2,4 | 0,2 | 0,1 |
| WX-532 | 0 | 0 | 0 |
| WX-538 | 0,2 | 0,2 | 0,1 |
| WX-550 | 0 | 3,1 | 0,6 |
| WX-582 | 0,4 | 3,1 | 5,2 |

| | | | |
|---|---|---|---|
| *... Vergleichsbeispiel | | | |

### 16.1.3 Ergebnisse

Caco-2-Zellmonoschichten bilden das humane intestinale absorptive Epithel nach und stellen ein wertvolles Werkzeug zur Untersuchung des transepithelialen Transports dar. Die TEER-Werte stellen eine Kontrolle für die Lebensfähigkeit und Zusammenhängigkeit der Monoschicht dar und waren zwischen 100 % und 120 %. TEER ist definiert als das Produkt von Widerstand x Oberfläche. Der Widerstand reflektiert die Resistivität über dichte Verbindungen (parazelluläre Route) und nicht über Zellmembranen (transzelluläre Route).

Die in dieser in vitro Transportuntersuchung erhaltenen Werte zeigen, dass für die untersuchten Verbindungen ein Transport von der apicalen Seite der epithelialen Zellschicht zur basolateralen Seite auftritt. Die Ergebnisse zeigen, dass die untersuchten Verbindungen eine Bioverfügbarkeit aufweisen.

Die Ergebnisse für die untersuchten Verbindungen sind nochmals in der folgenden Tabelle zusammengefasst:

**Tabelle: Bestimmung der Inhibitorischen Konstante, Spezifität und der oralen Bioverfügbarke**

| Formel | Bez. | Ki [µM] uPA | Ki [µM] Plasmin | Ki [µM] FXa | Ki [µM] Thrombin | Ki [µM] PK | Caco-2 assay % basolateral |
|---|---|---|---|---|---|---|---|
| 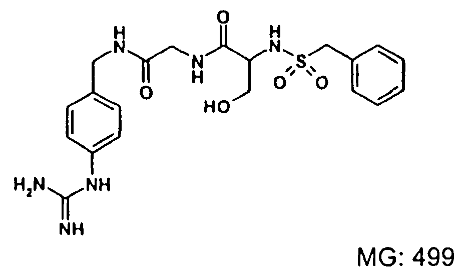 | **WX-508** | **0,034** | >200 | | > 350 | 180 | 0,2 / 0,1/ 0,7 |
| 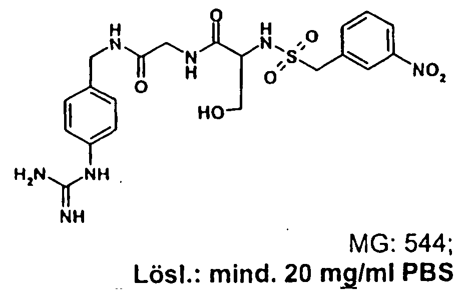 | **WX- C316** | **0,09** | no inh. at 100 µM *) | 32.4 | no inh. at 100 µM *) | 875 | 0,2 / 0,2 / 3,4 0,1 / 0,1/ 3,6 |
| 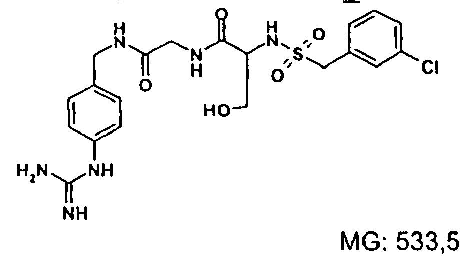 | **WX-** C318 | **0,047** | no inh. at 100 µM *) | 36 | no inh. at 100 µM *) | 176 | 0,4/1,0/4,5 0,2/0,6/7,0 |
| 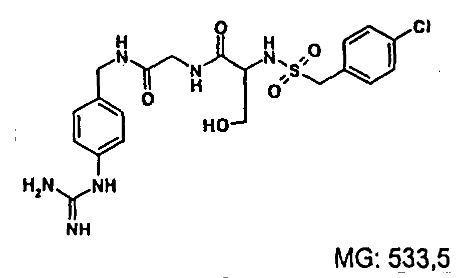 | **WX- C340** | **0,01** | no inh. at 100 µM *) | 34 | no inh. at 100 µM *) | 216 | 2,4/0,2/0,1 2,3/0,1/0,1 |
| 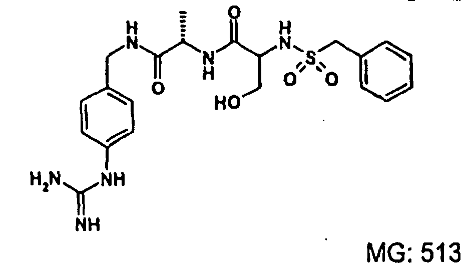 | **WX-532** | **0,016** | 40 | | no inh. at 100 µM *) | 49 | 0/0/0 |
| 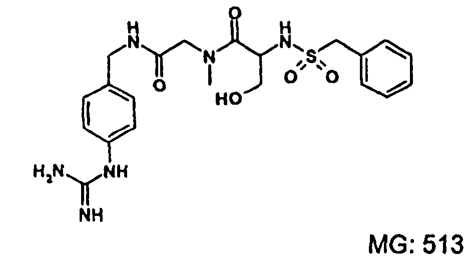 | **WX-538 N-Me** | **0,041** | 130 | >100 | > 100 | 109 | 0,2 / 0,2 / 0,1 0,1 / 0,1 / 0 |
| 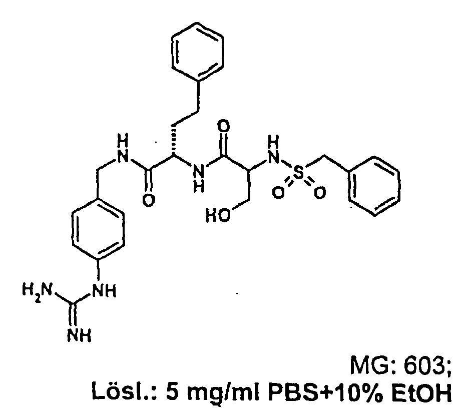 | **WX-550 Homo- Phe** | **0,10** | **2,5** | >100 | >50 | 268 | 0/3,1/0,6 |
| 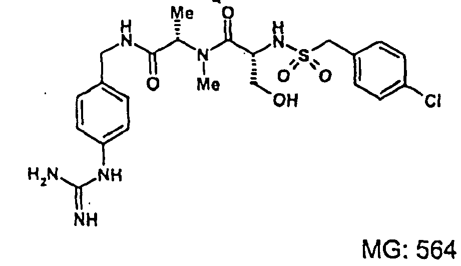 | **WX-582** | **0,01** | | | | 58 | 0 / 3,1 / 5,2 0,4 / 2,9 / 3,0 |
| 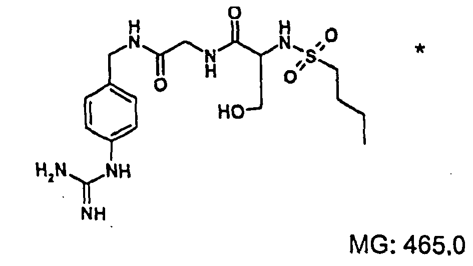 | **WX- C300** | **0,27** | | | | | |
| 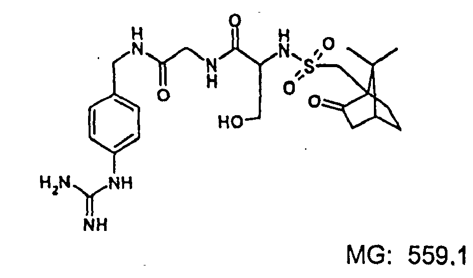 | **WX-C298 *** | **0,16** | | | | | |
| | **WX-C324 *** | | | | | | 0,1 / 0,1 / 2,9 0 / 0 / 3,0 |

| | | | | | | | |
|---|---|---|---|---|---|---|---|
| * Vergleichsbeispiel | | | | | | | |

### Beispiel 17: In vivo Untersuchungen

Zehn erfindungsgemäße uPA Inhibitor-Kandidaten wurden in Nagern hinsichtlich oraler Bioverfügbarkeit und Plasmakinetik untersucht. Hierzu wurden Versuche an neun Wochen alten weiblichen Balb/c-Mäusen mit einem Gewicht von 17,6 bis 18,5 g durchgeführt. Die folgenden Testsubstanzen, welche Guanidinophenylalanin-Derivate mit HCl als Gegenion sind, wurden eingesetzt:

| | | | MW einschl. HCl | Ki human uPA [µM] |
|---|---|---|---|---|
| 1 | | WX-508 | 499 | 0,034 |
| 2 | | WXC-316 | 544 | 0,09 |
| 3 | | WXC-318 | 534 | 0,047 |
| 4 | | WXC-340 | 533 | 0,01 |
| 5 | | WX-532 | 513 | 0,016 |
| 6 | | WX-538 | 513 | 0,041 |
| 7 | | WX-550- Homo-Phe | 603 | 0,10 |
| 8 | | WX-582 | 564 | 0,01 |
| 9 | | WXC-300 * | 465 | 0,27 |
| 10 | | WXC-298 * | 559 | 0,016 |

| | | | | |
|---|---|---|---|---|
| * ... **Vergleichsbeispiel** | | | | |

Die Verbindungen wurden im Bereich von 0,1 bis 1000 mg/kg, insbesondere 0,5 bis 500 mg und mehr bevorzugt 3 bis 300 mg/kg eingesetzt. Die Verabreichung erfolgte dabei über eine Magensonde. Die Probenahme erfolgte bei 20, 40 und 60 Minuten.

Die Ergebnisse sind in der folgenden Tabelle zusammengefasst.

| Test-substanz | Dosis mg/kg | Zeitpunkt der Probennahme | c [µg/ml] im Serum 1.Analyse | c [µg/ml] im Serum 2. Analyse |
|---|---|---|---|---|
| WX-508 | 10 i.v. | 0 | 41,1 | 55,5 |
| | | 10 | 4,3 | 4,7 |
| | | 30 | 0,7 | 0,2 |
| | 30 oral | 20 | 0,3 | 0,3 |
| | | 40 | 5,7 | 4,5 |
| | | 60 | 0,08 | 0,09 |
| | 300 oral | 20 | 3,5 | - |
| | | 40 | 7,5 | 8,3 |
| | | 60 | 5,4 | 5,8 |
| WXC-316 | 10 i.v. | 0 | 45,4 | 67,5 |
| | | 10 | 12,4 | 14,3 |
| | | 30 | 2,7 | 2,6 |
| | 30 oral | 20 | 0,5 | 0,6 |
| | | 40 | 5,9 | 7,1 |
| | | 60 | 0,4 | 0,5 |
| | 300 oral | 20 | 6,0 | 7,2 |
| | | 40 | 21,8 | 32,1 |
| | | 60 | 5,3 | 5,8 |
| WXC-318 | 10 i.v. | 0 | - | 48,4 |
| | | 10 | 4,3 | 5,5 |
| | | 30 | 0,7 | 1,4 |
| | 30 oral | 20 | 0,1 | 0,2 |
| | | 40 | - | 0,05 |
| | | 60 | 0,2 | 0,2 |
| | 300 oral | 20 | 100 | 82,6 |
| | | 40 | 5,4 | 6,2 |
| | | 60 | 7,5 | 7,7 |
| WXC-340 | 10 i.v. | 0 | 51,8 | |
| | | 10 | 5,3 | |
| | | 30 | 1,7 | |
| | 30 oral | 20 | 0,3 | 0,5 |
| | | 40 | 0,3 | 0,8 |
| | | 60 | 1,4 | 2,3 |
| | 300 oral | 20 | 4,5 | |
| | | 40 | 4,9 | |
| | | 60 | 4,3 | |
| WX-532 | 10 i.v. | 0 | 61,7 | 68,8 |
| | | 10 | 3,8 | 3,5 |
| | | 30 | 11,4 | 11,2 |
| | 30 oral | 20 | 1,5 | |
| | | 40 | 10,9 | |
| | | 60 | 0,9 | |
| | 300 oral | 20 | 5,8 | 6,7 |
| | | 40 | 2,9 | 3,3 |
| | | 60 | 12,8 | 15,0 |
| WX-538 | 10 i.v. | 0 | 45,8 | |
| | | 10 | 5,5 | |
| | | 30 | 0,6 | |
| | 30 oral | 20 | 0,1 | |
| | | 40 | 0,05 | |
| | | 60 | 0,2 | |
| | 300 oral | 20 | 2,5 | |
| | | 40 | 3,5 | |
| | | 60 | 2,3 | |
| WX-550 | 2,5 i.v. | 0 | 7,2 | |
| | | 10 | 0,7 | |
| | | 30 | 0,02 | |
| | 7,5 oral | 20 | 0 | |
| | | 40 | 0 | |
| | | 60 | 0 | |
| | 75 oral | 20 | 0 | |
| | | 40 | 0,3 | |
| | | 60 | 0 | |
| WX-582 | 2,5 i.v. | 0 | 11,5 | |
| | | 10 | 0,8 | |
| | | 30 | 0,1 | |
| | 7,5 oral | 20 | 0 | |
| | | 40 | 0 | |
| | | 60 | 0 | |
| | 75 oral | 20 | 1 | |
| | | 40 | 0,7 | |
| | | 60 | 0,4 | |
| WXC-298 * | 10 i.v. | 0 | 66,2 | |
| | | 10 | 18,3 | |
| | | 30 | 2,1 | |
| | 30 oral | 20 | 0,2 | |
| | | 40 | 0,4 | |
| | | 60 | 0,1 | |
| | 300 oral | 20 | 17,0 | |
| | | 40 | 10,7 | |
| | | 60 | 7,0 | |
| WXC-300 * | 10 i.v. | 0 | 94,4 | |
| | | 10 | 11,0 | |
| | | 30 | 1,3 | |
| | 30 oral | 20 | 0,2 | |
| | | 40 | 0,2 | |
| | | 60 | 0,2 | |
| | 300 oral | 20 | 1,5 | |
| | | 40 | 4,6 | |
| | | 60 | 0,6 | |

| | | | | |
|---|---|---|---|---|
| * Vergleichsbeispiel | | | | |

## Patentansprüche

1. N-[2-(4-Guanidino-benzolsulfonylamino)-ethyl]-3-hydroxy-2-phenylmethansulfonylamino-propionamid Hydrochlorid (WX-568), Bz-SO₂-(D)-Ser-(Aza-Gly)-4-Guanidino-benzylamid Hydrochlorid (WX-544), N-(4-Guanidino-benzyl)-2-(3-hydroxy-2-phenylmethansulfonylamino-propionylamino)-4-phenyl-butyramid Hydrochlorid (WX-550), 3-Nitrobenzyl-sulfonyl-(D)-Ser-Gly-(4-Guanidinobenzyl) amid Hydrochlorid (WX-C316), Benzylsulfonyl-(D)-Ser-N-Me-Gly-(4-Guanidinobenzyl)amid (WX-538), N-[(4-Guanidino-benzylcarbamoyl)-methyl]-3-hydroxy-2-phenylmethansulfonylaminopropionamid (WX-508), 4-Chlorbenzylsulfonyl-(d)-Ser-N-Me-Ala-(4-Guanidinobenzyl) amid (WX-582), 4-Chlorbenzylsulfonyl-(D)-Ser-Gly-(4-Guanidinobenzyl)amid Hydrochlorid (WX-C340), 3-Chlorbenzylsulfonyl-(D)-Ser-Gly-(4-Guanidinobenzyl)amid Hydrochlorid (WX-C318).

2. Verwendung der Verbindungen N-[2-(4-Guanidino-benzolsulfonylamino)-ethyl]-3-hydroxy-2-phenyl-methansulfonylamino-propionamid Hydrochlorid (WX-568), Bz-SO₂-(D)-Ser-(Aza-Gly)-4-Guanidinobenzylamid Hydrochlorid (WX-544), N-(4-Guanidino- benzyl)-2-(3-hydroxy-2-phenylmethan-sulfonylamino-propionylamino)-4-phenylbutyramid Hydrochlorid (WX-550), 3-Nitrobenzyl-sulfonyl-(D)-Ser-Gly-(4-Guanidinobenzyl)amid Hydrochlorid (WX-C316), Benzylsulfonyl-(D)-Ser-N-Me-Gly-(4-Guanidinobenzyl)amid (WX-538), N-[(4-Guanidino-benzylcarbamoyl)-methyl]-3-hydroxy-2-phenylmethansulfonylaminopropionamid (WX-508), 4-Chlorbenzylsulfonyl-(d)-Ser-N-Me-Ala-(4-Guanidinobenzyl)amid (WX-582), Benzylsulfonyl-(D)-Ser-Ala-(4-Guanidinobenzyl)amid Hydrochlorid (WX-532), 4-Chlorbenzylsulfonyl-(D)-Ser-Gly-(4-Guanidinobenzyl)amid Hydrochlorid (WX-C340), 3-Chlorbenzyl-sulfonyl-(D)-Ser-Gly-(4-Guanidinobenzyl)amid Hydrochlorid (WX-C318) oder Salzen dieser Verbindungen zur Herstellung eines Mittels zur selektiven Hemmung des Urokinase-Plasminogenaktivators.

3. Verwendung nach Anspruch 2 zur Herstellung eines Medikaments zur Bekämpfung von Krankheiten, die mit einer pathologischen Überexpression von Urokinase oder/und Urokinaserezeptor assoziiert sind.

4. Verwendung nach einem der Ansprüche 2 oder 3 zur Herstellung eines Medikaments zur Tumorbekämpfung.

5. Verwendung nach einem der Ansprüche 2 bis 4 zur Herstellung eines Medikaments zur Bekämpfung der Metastasenbildung.

6. Verwendung nach einem der Ansprüche 2 bis 5 zur Herstellung eines Medikaments, welches oral, topisch, rektal, parenteral, subkutan, intramuskulär, intraperitoneal, sublingual, nasal oder inhalativ verabreichbar ist.

7. Verwendung nach einem der Ansprüche 2 bis 6, worin das Mittel in Form von Tabletten, Dragees, Kapseln, Pellets, Suppositoren, Lösungen, Emulsionen, Suspensionen, Liposomen, Inhalationssprays oder transdermalen Systemen, wie Pflastern, hergestellt wird.

8. Verwendung einer Verbindung nach Anspruch 1 zur Herstellung eines Medikaments zur Urokinasehemmung bei Lebewesen.

9. Verwendung nach Anspruch 8 zur Herstellung eines Medikaments zur Urokinasehemmung beim Menschen.

10. Arzneimittel enthaltend eine therapeutisch aktive Menge einer Verbindung nach Anspruch 1.

## Claims

1. N-[2-(4-Guanidino-benzenesulfonyl-amino)-ethyl]-3-hydroxy-2-phenylmethane-sulfonylamino-propionamide hydrochloride (WX-568), Bz-SO₂-(D)-Ser-(Aza-Gly)-4-guanidino-benzylamide hydrochloride (WX-544), N-(4-guanidino-benzyl)-2-(3-hydroxy-2-phenylmethane-sulfonylaminopropionylamino)-4-phenyl-butyramide hydrochloride (WX-550), 3-nitrobenzyl-sulfonyl-(D)-Ser-Gly-(4-guanidinobenzyl)amide hydrochloride (WX-C316), benzylsulfonyl-(D)-Ser-N-Me-Gly-(4-guanidinobenzyl)amide (WX-538), N-[(4-guanidino-benzylcarbamoyl)-methyl]-3-hydroxy-2-phenylmethanesulfonylaminopropionamide (WX-508), 4-chlorobenzylsulfonyl-(D)-Ser-N-Me-Ala-(4-guanidinobenzyl)amide (WX-582), 4-chlorobenzylsulfonyl-(D)-Ser-Gly-(4-guanidinobenzyl)amide hydrochloride (WX-C340), 3-chlorobenzylsulfonyl-(D)-Ser-Gly-(4-guanidinobenzyl)amide hydrochloride (WX-C318).

2. Use of the compounds N-[2-(4-guanidino-benzenesulfonyl-amino)-ethyl]-3-hydroxy-2-phenylmethane-sulfonylamino-propionamide hydrochloride (WX-568), Bz-SO₂-(D)-Ser-(Aza-Gly)-4-guanidino-benzylamide hydrochloride (WX-544), N-(4-guanidino-benzyl)-2-(3-hydroxy-2-phenylmethanesulfonylamino-propionylamino)-4-phenyl-butyramide hydrochloride (WX-550), 3-nitrobenzyl-sulfonyl-(D)-Ser-Gly-(4-guanidinobenzyl)amide hydrochloride (WX-C316), benzylsulfonyl-(D)-Ser-N-Me-Gly-(4-guanidinobenzyl)amide (WX-538), N-[(4-guanidino-benzylcarbamoyl)-methyl]-3-hydroxy-2-phenylmethanesulfonylaminopropionamide (WX-508), 4-chlorobenzylsulfonyl-(D)-Ser-N-Me-Ala-(4-guanidinobenzyl)amide (WX-582), benzylsulfonyl-(D)-Ser-Ala-(4-guanidinobenzyl)amide hydrochloride (WX-532), 4-chlorobenzylsulfonyl-(D)-Ser-Gly-(4-guanidinobenzyl)amide hydrochloride (WX-C340), 3-chlorobenzyl-sulfonyl-(D)-Ser-Gly-(4-guanidinobenzyl)amide hydrochloride (WX-C318) or salts of these compounds to produce an agent for the selective inhibition of the urokinase plasminogen activator.

3. Use as claimed in claim 2 to produce a pharmaceutical preparation to combat diseases that are associated with a pathological overexpression of urokinase or/and urokinase receptor.

4. Use as claimed in one of the claims 2 or 3 to produce a pharmaceutical preparation to combat tumours.

5. Use as claimed in one of the claims 2 to 4 to produce a pharmaceutical preparation to combat the formation of metastases.

6. Use as claimed in one of the claims 2 to 5 to produce a pharmaceutical preparation that can be administered orally, topically, rectally, parenterally, subcutaneously, intramuscularly, intraperitoneally, sublingually, nasally or by inhalation.

7. Use as claimed in one of the claims 2 to 6, wherein the agent is produced in the form of tablets, dragees, capsules, pellets, suppositories, solutions, emulsions, suspensions, liposomes, inhalation sprays or transdermal systems such as plasters.

8. Use of a compound as claimed in claim 1 to produce a pharmaceutical preparation to inhibit urokinase in living organisms.

9. Use as claimed in claim 8 to produce a pharmaceutical preparation to inhibit urokinase in humans.

10. Pharmaceutical preparation containing a therapeutically-active amount of a compound as claimed in claim 1.

## Revendications

1. Chlorhydrate de N-[2-(4-guanidinobenzènesulfonylamino)éthyl]-3-hydroxy-2-phénylméthanesulfonylaminopropionamide (WX-568), chlorhydrate de Bz-SO₂-(D)-Ser-(Aza-Gly)-4-guanidinobenzylamide (WX-544), chlorhydrate de N-(4-guanidinobenzyl)-2-(3-hydroxy-2-phénylméthanesulfonylaminopropionylamino)-4-phénylbutyramide (WX-550), chlorhydrate de 3-nitrobenzylsulfonyl-(D)-Ser-Gly-(4-guanidinobenzyl)amide (WX-C316), benzylsulfonyl-(D)-Ser-N-Me-Gly-(4-guanidinobenzyl)amide (WX-538), N-[(4-guanidinobenzylcarbamoyl)méthyl]-3-hydroxy-2-phénylméthanesulfonylaminopropionamide (WX-508), 4-chlorobenzylsulfonyl-(D)-Ser-N-Me-Ala-(4-guanidinobenzyl)amide (WX-582), chlorhydrate de 4-chlorobenzylsulfonyl)-(D)-Ser-Gly-(4-guanidinobenzyl)amide (WX-C340), chlorhydrate de 3-chlorobenzylsulfonyl)-(D)-Ser-Gly-(4-guanidinobenzyl)amide (WX-C318).

2. Utilisation des composés chlorhydrate de N-[2-(4-guanidinobenzènesulfonylamino)éthyl]-3-hydroxy-2-phénylméthanesulfonylaminopropionamide (WX-568), chlorhydrate de Bz-SO₂-(D)-Ser-(Aza-Gly)-4-guanidinobenzylamide (WX-544), chlorhydrate de N-(4-guanidinobenzyl)-2-(3-hydroxy-2-phénylméthanesulfonylamino-propionylamino)-4-phénylbutyramide (WX-550), chlorhydrate de 3-nitrobenzylsulfonyl-(D)-Ser-Gly-(4-guanidinobenzyl)amide (WX-C316), benzylsulfonyl-(D)-Ser-N-Me-Gly-(4-guanidinobenzyl)amide (WX-538), N-[(4-guanidinobenzylcarbamoyl)méthyl]-3-hydroxy-2-phénylméthanesulfonylamino-propionamide (WX-508), 4-chlorobenzylsulfonyl-(D)-Ser-N-Me-Ala-(4-guanidinobenzyl)amide (WX-582), chlorhydrate de benzylsulfonyl-(D)-Ser-Ala-(4-guanidinobenzyl)amide (WX-532), chlorhydrate de 4-chlorobenzylsulfonyl)-(D)-Ser-Gly-(4-guanidinobenzyl)amide (WX-C340), chlorhydrate de 3-chlorobenzylsulfonyl)-(D)-Ser-Gly-(4-guanidinobenzyl)amide (WX-C318) ou de sels de ces composés pour la préparation d'une composition destinée à l'inhibition sélective de l'activateur du plasminogène de type urokinase.

3. Utilisation selon la revendication 2 pour la préparation d'un médicament destiné à combattre des maladies qui sont associées à une surexpression pathologique d'urokinase et/ou d'un récepteur d'urokinase.

4. Utilisation selon l'une des revendications 2 ou 3 pour la préparation d'un médicament destiné à lutter contre les tumeurs.

5. Utilisation selon l'une des revendications 2 à 4 pour la préparation d'un médicament destiné à lutter contre la formation de métastases.

6. Utilisation selon l'une des revendications 2 à 5 pour la préparation d'un médicament qui peut être administré par voie orale, topique, rectale, parentérale, sous-cutanée, intramusculaire, intrapéritonéale, sublinguale, nasale ou par inhalation.

7. Utilisation selon l'une des revendications 2 à 6, dans laquelle la composition est sous forme de comprimés, de dragées, de capsules, de pastilles, de suppositoires, de solutions, d'émulsions, de suspensions, de liposomes, de pulvérisations à inhaler ou de systèmes transdermiques comme des pansements.

8. Utilisation d'un composé selon la revendication 1 pour la préparation d'un médicament destiné à inhiber l'urokinase chez un être vivant.

9. Utilisation selon la revendication 8 pour la préparation d'un médicament destiné à inhiber l'urokinase chez l'homme.

10. Médicament contenant une quantité thérapeutiquement active d'un composé selon la revendication 1.
